# EUROPÄISCHE PATENTSCHRIFT

(11) **EP 2 130 493 B1**
(45) Veröffentlichungstag und Bekanntmachung des Hinweises auf die Patenterteilung: **25.09.2013**
(21) Anmeldenummer: 08010403.7
(22) Anmeldetag: 07.06.2008
(51) Int. Cl.: A61B 5/15, A61B 5/145, A61B 5/157, A61B 5/151, A61B 5/1455

(54) **Analysesystem zur Bestimmung eines Analyten in einer Körperflüssigkeit, Magazin für ein Analysegerät und Verfahren zur Herstellung eines Magazins für ein Analysegerät.**
Analysis system for detecting an analyte in a bodily fluid, cartridge for an analytic device and method for manufacturing a cartridge for an analysis system.
Système d'analyse destiné à la détermination d'un analyte dans un liquide corporel, cartouche pour un appareil d'analyse et procédé de production d'une cartouche pour un appareil d'analyse.

(43) Veröffentlichungstag der Anmeldung: 09.12.2009
(73) Patentinhaber: Roche Diagnostics GmbH, 68305 Mannheim (DE); F.Hoffmann-La Roche AG, 4070 Basel (CH)
(72) Erfinder: Frey, Stephan-Michel, 64347 Griesheim (DE); List, Hans, 64754 Hessenech-Kailbach (DE); Rittinghaus, Andrea, 69239 Neckarsteinach (DE); Zimmer, Volker, 67229 Laumersheim (DE); Ihle, Günther, 69256 Mauer (DE); Roedel, Wolfgang, 69123 Heidelberg (DE)
(74) Vertreter: Petirsch, Markus

(56) Entgegenhaltungen:
- WO-A-02/00101
- WO-A-2007/077212
- US-A1- 2003 199 789
- US-A1- 2004 039 303
- US-A1- 2004 098 009
- US-A1- 2007 219 462

## Beschreibung

Die vorliegende Erfindung betrifft ein Analysesystem zur Bestimmung eines Analyten in einer durch einen Stich in die Haut gewonnenen Körperflüssigkeitsprobe, umfassend ein Magazin mit Kammern und ein wiederverwendbares Analysegerät. Die Kammern des Magazins enthalten Analyseelemente mit einer Probenkontaktzone und einem mindestens ein Reagenz enthaltenen Reagenzsystem, dessen Reaktion mit einer Körperflüssigkeit zu einer messbaren Änderung einer Messgröße führt, und Stechelemente mit einer Spitze zum Einstechen in die Haut mit einem Kapillarkanal, der eine Fluidverbindung zwischen der Spitze und einer Probenübergabezone des Stechelementes bildet. Das Analysegerät hat einen Stechantrieb zum Antreiben einer Stechbewegung eines Stechelementes auf einem Bewegungsweg, eine Halterung zur Aufnahme des Magazins und eine Mess- und Auswerteeinrichtung zur Messung der messbaren Änderung einer Messgröße zur Ermittlung eines gewünschten Analyseergebnisses. Die Erfindung betrifft ebenfalls ein Verfahren zur Herstellung eines zweiteiligen Magazins mit einem Stechelement-Teilmagazin mit Stechelementkammern und einem Analyseelement-Teilmagazin mit Analyseelementkammern.

Die Erfindung betrifft auch ein Magazin mit Kammern für ein Analysegerät. Die Kammern des Magazins enthalten Analyseelemente mit einer Probenkontaktzone und mit einem mindestens ein Reagenz enthaltenden Reagenzsystem sowie Stechelemente mit einer Spitze zum Einstechen in die Haut mit einem Kapillarkanal.

Auf vielen Gebieten der medizinischen Analyse werden Analyten in einer Körperflüssigkeitsprobe, insbesondere Blut, bestimmt, die durch einen Stich in die Haut eines Patienten gewonnen wird. Bei der Entnahme der Körperflüssigkeit aus einem Körperteil, vorzugsweise aus der Fingerbeere, reichen geringe Mengen der Körperflüssigkeit aus, um einen Analyten, wie beispielsweise den Glucosegehalt, für medizinische und diagnostische Zwecke zu bestimmen. Die eingesetzten Geräte sind so konstruiert, dass sie nicht nur von medizinischem Fachpersonal, sondern auch von Laien problemlos verwendet werden können.

Um einen Analyten in einer aus einem Körperteil austretenden Körperflüssigkeitsprobe zu bestimmen, sind zwei Schritte notwendig. Zuerst muss durch einen Stich in die Haut eines Körperteils eine Wunde erzeugt werden, was beispielsweise mittels eines Stechgerätes erfolgen kann. In einem zweiten Schritt wird die austretende Körperflüssigkeit aufgenommen und in einem Analysegerät analysiert. Die Verwendung von zwei unabhängig voneinander arbeitenden Geräten ist für den Benutzer unkomfortabel und umständlich. Deshalb wurden kombinierte Systeme entwickelt, die die beiden bisherigen Geräte zu einem Gerät vereinen und beide Schritte ausführen können. Die bekannten Geräte sind jedoch relativ groß.

Neue Entwicklungen umfassen integrierte Analysesysteme. Die in der Regel automatisierten Geräte ermöglichen ein "One-Step-Treatment", so dass der Benutzer das System nur einmal ansetzen muss und das Analyseergebnis - ohne weitere Handlungsschritte - ablesen kann.

Bei einem ersten Typ solcher integrierter Systeme werden getrennte Stechelemente und Analyseelemente verwendet. Innerhalb eines kombinierten Stech- und Analysegerätes werden die erforderlichen Bewegungen zum Einstechen in die Haut und zur Übertragung eines dabei gewonnenen Probenflüssigkeitstropfens auf das Analyseelement mittels einer in das Gerät integrierten Bewegungsmechanik realisiert. Der Bluttransfer aus der erzeugten Wunde zu dem Analyseelement ist schwierig zu realisieren. Dabei ist zu berücksichtigen, dass moderne Geräte mit extrem kleinen Blutmengen arbeiten sollen. Weitere Probleme betreffen die Bewegungs- und Ankopplungsmechanik der Lanzetten und Stechelemente.

Beispielsweise beschreibt die WO 02/00101 A2 ein Analysesystem mit Hohlnadeln zum Flüssigkeitstransport aus einer Wunde in eine Flüssigkeitssammelkammer bevor die Flüssigkeit auf ein Analyseelement übertragen wird. Die US 2004/098009 A1 offenbart ein Analysesystem mit Stechelementen, bei dem die Stechelement in Kammern gelagert sind. Analyseelemente werden in einem Analyseelement-Teilmagazin in einzelnen Kammern gelagert und zum Blutübertrag aus den Kammern herausbewegt. Bevorzugt findet nach dem Einstich in ein Körperteil ein direkter Übertrag des Blutes aus der Wunde auf das Analyseelement statt. Alternativ können Stechelement und Analyseelement außerhalb ihrer Kammern so angenähert werden, dass ein Flüssigkeitsübertrag erfolgen kann.

Die Probleme mit dem Transport sehr kleiner Blutmengen werden vermindert und die Mechanik des Gerätes wird vereinfacht, wenn statt getrennter Stechelemente und Analyseelemente integrierte Probengewinnungs- und Analyseelemente eingesetzt werden, bei denen das Stechelement und das Analyseelement in einer disposiblen Einheit vereint sind. Wegen dieser Vorteile wurden in jüngerer Zeit überwiegend Systeme mit solchen integrierten Probengewinnungs- und Analyseelementen vorgeschlagen.

Beispiele sind in den folgenden Druckschriften beschrieben:
1) WO 2006/092281 A1
2) US 2003/0212345 A1
3) US 6,607,658 B1
4) US 2004/098009 A1

Es ist eine Aufgabe der vorliegenden Erfindung, die bekannten integrierten Systeme zu verbessern. Insbesondere sollen die verbesserten Systeme eine kompakte Baugröße aufweisen und kostengünstig herstellbar sein.

Gelöst wird das zugrundeliegende Problem durch ein Analysesystem mit den Merkmalen des Anspruchs 1, ein Magazin mit den Merkmalen des Anspruchs 2 und durch ein Verfahren zur Herstellung eines Magazins mit den Merkmalen des Anspruchs 14.

Die auf die unabhängigen Ansprüche rückbezogenen Unteransprüche definierten bevorzugte Weiterbildungen der Gegenstände der unabhängigen Ansprüche.

Das Analysesystem zur Bestimmung eines Analyten in einer durch einen Stich in die Haut gewonnen Körperflüssigkeitsprobe umfasst ein Magazin mit Kammern und ein wiederverwendbares Analysegerät mit einem Stechantrieb, einer Halterung zur Aufnahme des Magazins und einer Mess- und Auswerteeinrichtung.

Das Magazin enthält in seinen Kammern Analyseelemente mit einer Probenkontaktzone und einem mindestens ein Reagenz enthaltenen Reagenzsystem, dessen Reaktion mit der Körperflüssigkeit zur einer messbaren Änderung einer Messgröße führt, und Stechelemente mit einer Spitze zum Einstechen in die Haut mit einem Kapillarkanal, der eine Fluidverbindung zwischen der Spitze und einer Probenübergabezone des Stechelementes bildet.

Der Stechantrieb des Analysesystems treibt eine Stechbewegung eines Stechelements auf einem Bewegungsweg an, der eine Vortriebsphase in Einstichrichtung und nach Erreichen eines Umkehrpunktes der Stechbewegung eine Rückführungsphase entgegen der Einstichrichtung umfasst. In der Halterung des Analysegerätes kann das Magazin derart aufgenommen werden, dass sich jeweils eine Kammer des Magazins in einer Funktionsposition befindet, in der ein Stechelement in der Kammer von dem Stechantrieb bewegt werden kann. Die Mess- und Auswerteeinrichtung ist zur Messung der messbaren Änderung einer Messgröße und zur Ermittlung des gewünschten Analyseergebnisses eingerichtet.

Das Magazin umfasst zwei Teilmagazine, nämlich ein StechelementTeilmagazin mit Stechelementkammern, die jeweils ein Stechelement enthalten, und ein Analyseelement-Teilmagazin mit Analyseelementkammem, die jeweils ein Analyseelement enthalten. Somit sind die Stechelemente und die Analyseelemente in getrennten Kammem - zumindest im Auslieferungszustand des Magazins, bevorzugt immer - enthalten.

Die Stechelementkammer weist eine Stechelementaustrittsöffnung auf. Die Analyseelementkammer hat eine Stechelementeintrittsöffnung, die mittels einer Verschlussfolie verschlossen ist. Wenn sich das Magazin in der Funktionsposition befindet, sind die beiden Teilmagazine relativ zueinander so angeordnet oder können in eine solche Position gebracht werden, dass jeweils eine Stechelementaustrittsöffnung einer Stechelementkammer und eine Stechelementeintrittsöffnung einer Analyseelementkammer so benachbart sind, dass die beiden Öffnungen fluchten und ein Stechelement aus der Stechelementkammer durch die Stechelementaustrittsöffnung und die Stechelementeintrittsöffnung in die benachbarte Analysekammer bewegt werden kann. Die Verschlussfolie, die die Stechelementeintrittsöffnung der Analyseelementkammer zumindest im Lieferzustand verschließt, wird vor oder während der Stechbewegung des Stechelementes aus der Stechelementkammer in die Analyseelementkammer geöffnet, z.B. durchstochen oder durchstoßen. Im Folgenden wird ohne Beschränkung der Allgemeinheit durchstoßen als öffnen verwendet.

Das Aufstehen und Durchstoßen der Verschlussfolie kann bevorzugt durch das Stechelement selbst geschehen. Die Folie kann aber auch durch ein anderes Element geöffnet werden, bevor das Stechelement aus der Stechelementaustrittsöffnung austritt.

Das zweiteilige Magazin mit Stechelement-Teilmagazin und Analyseelement-Teilmagazin hat den Vorteil, dass den unterschiedlichen und teilweise gegenläufigen Anforderungen der Analyseelemente und Stechelemente Rechnung getragen werden kann. Das Stechelement kann separat sterilisiert werden, beispielsweise mittels Beta-Strahlung, ohne dass auch die Analyseelemente der Strahlung ausgesetzt werden. Zum Beispiel kann das Stechelement-Teilmagazin vor dem Zusammenbau mit dem Analyseelement-Teilmagazin mit Stechelementen bestückt werden. Die Stechelementaustrittsöffnung wird bevorzugt mit einer Sterilhaltefolie verschlossen, so dass die Stechelementkammern verschlossen sind. Das Verschließen wird bevorzugt vor der Sterilisierung des Stechelement-Teilmagazins und nach dem Bestücken der Stechelementkammem mit einem Stechelement ausgeführt. Anschließend wird das Stechelement-Teilmagazin der Beta-Strahlung ausgesetzt, so dass alle Stechelemente sterilisiert werden. Da das Analyseelement mit den enthaltenen Reagenzien in einem getrennten Teilmagazin enthalten sind, werden sie bei der Sterilisierung des Stechelement-Teilmagazins nicht durch die Strahlung geschädigt.

Erfindungsgemäß schließt der Bewegungsweg des Stechelementes eine Übergabeposition innerhalb der Kammer ein, in der die Probenübergabezone des Stechelementes der Probenkontaktzone des Analyseelementes benachbart ist, um eine Fluidverbindung zur Übertragung einer Körperflüssigkeitsprobe von dem Stechelement an das Analyseelement herzustellen. Ein Fluidkontakt zwischen dem Stechelement und dem Analyseelement findet folglich ausschließlich in der Übergabeposition des Stechelements statt, in der es sich in der Analyseelementkammer befindet. In anderen Positionen des Stechelementes auf seinem Bewegungsweg kann kein Flüssigkeitstransport an das Analyseelement geschehen. Auf diese Weise kann ein exakt gesteuerter Flüssigkeitsübertrag erfolgen. Hierbei ist neben der örtlichen Steuerung auch eine zeitliche Steuerung möglich. Beispielsweise kann das Stechelement in der Übergabeposition eine vorgegebene Zeit verharren, beispielsweise um einen Fluidtransport in die Probenübergabezone des Stechelements zu ermöglichen, bevor das Stechelement und das Analyseelement derart benachbart sind, dass der Probenübertrag stattfindet. Ein kraftgesteuerter Blutübertrag ist in einer bevorzugten Ausführungsform ebenfalls möglich.

Vorzugsweise werden hierzu das Stechelement und das Analyseelement relativ aufeinander zubewegt, bis sie bevorzugt aneinander kontaktieren. Die Relativbewegung zwischen den beiden Elementen kann durch eine Querbewegung (in Bezug zur Einstichrichtung) eines der beiden Elemente erfolgen, bevorzugt durch eine Querbewegung des Stechelements.

In einer bevorzugten Ausführungsform wird die Relativbewegung zwischen dem Analyseelement und dem Stechelement durch eine Schwenkbewegung des Verbindungselementes bewirkt, das mit dem Stechelement gekoppelt ist und eine Verbindung zwischen dem Stechelement und dem Stechantrieb herstellt. Dabei erstreckt sich das Verbindungselement wenigstens teilweise in die Stechelementkammer. Die Schwenkbewegung des Verbindungselements kann beispielsweise durch eine Steuerkurve des Antriebsmechanismus verwirklicht sein. Ebenso ist eine Bewegung des Verbindungselements quer zur Stecheinrichtung denkbar.

Erfindungsgemäß werden bei der Herstellung des zweiteiligen Magazins mehrere Schritte ausgeführt, deren Abfolge von der hier angegebenen Reihenfolge abweichen kann oder bei der einzelne Schritte zusammengefasst und/oder gemeinsam abgearbeitet werden können.

Zu Beginn des Herstellungsprozesses sind die beiden Teilmagazine voneinander getrennt. Das Stechelement-Teilmagazin wird mit Stechelementen bestückt. Sowohl die Stechelementaustrittsöffnung als auch die Verbindungselementeintrittsöffnung jeder Stechelementkammer werden jeweils mit einer Verschlussfolie verschlossen. Die in dem Stechelement-Teilmagazin enthaltenen Stechelemente werden in einem weiteren Schritt sterilisiert. In einem anderen Schritt werden die beiden Teilmagazine miteinander verbunden oder gekoppelt. Die Teilmagazine können relativ zueinander beweglich verbunden sein. Bevorzugt werden die Teilmagazine derart miteinander verbunden, dass eine relative Rotationsbewegung zwischen ihnen ausgeschlossen ist. Die Verschlussfolie der Stechelementaustrittsöffnung kann auch die Stechelementeintrittsöffnung des Analyseelement-Teilmagazins verschließen.

Ein weiterer Verfahrensschritt umfasst das Verschließen der Analyseelementkammern des Analyseelement-Teilmagazins an ihrer Stechelementeintrittsöffnung und an ihrer Magazinauslassöffnung jeweils mit einer Verschlussfolie. Ein anderer Herstellungsschritt sieht das Bestücken des Analyseelement-Teilmagazins mit Analyseelementen vor. Das Bestücken wird vor dem kompletten Verschließen der Analyseelementkammern oder vor dem Verschließen einer der Öffnungen durchgeführt. Bevorzugt werden die Analyseelemente in einer Halterung in den Kammern positioniert.

Die einzelnen Herstellungsschritte können in ihrer Reihenfolge variieren, insbesondere können beide Teilmagazine vor ihrer Verbindung bestückt werden. Wichtig bei der Herstellung ist nur, dass die Sterilisierung des Stechelement-Teilmagazins stattfindet, so lange das Analyseelement-Teilmagazin noch getrennt ist.

In einer bevorzugten Ausführung des Verfahrens findet das Verschließen der Stechelementeintrittsöffnung jeder Analyseelementkammer mit einer Verschlussfolie statt, bevor die beiden Teilmagazine miteinander verbunden werden. Bevorzugt werden beide Teilmagazine getrennt voneinander bestückt und verschlossen und erst anschließend zusammengebaut.

In einer bevorzugten Ausgestaltung des Verfahrens wird die Rotationsfestigkeit der beiden Teilmagazine erst beim Einsatz des Magazins in das Analysesystem bzw. das wiederverwendbare Analysegerät hergestellt.

Bei einteiligen Magazinen, die eine gemeinsame Kammer für Stechelement und Analyseelement aufweisen, kann das Problem, dass die zur Sterilisierung verwendete Strahlung die Reagenzien des Analyseelementes schädigt und teilweise unbrauchbar macht, dadurch gelöst, dass die Reagenzmenge in dem Analyseelement erhöht wird. Bei der Sterilisierung verbleibt dann eine ausreichende Menge funktionstüchtiger Reagenzien in dem Analyseelement, so dass eine Bestimmung eines Analyten in der Flüssigkeitsprobe durchgeführt werden kann. Die Verwendung einer erhöhten Reagenzmenge führt jedoch zu erheblichen Mehrkosten.

Ein weiterer Vorteil getrennter Teilmagazine besteht darin, dass keine Reagenzien des Reagenzsystems der Analyseelemente mit den Stechelementen in Kontakt kommen können, insbesondere nicht während der Herstellung oder Lagerung. Da die Reagenzien nicht in die Haut gelangen dürfen, ist es wichtig, dass die Stechelemente nicht kontaminiert werden.

Neben der Möglichkeit der getrennten Herstellung und Sterilisierung weist das erfindungsgemäße Analysesystem den Vorteil auf, dass die beiden Teilmagazine für die jeweiligen Anforderungen optimiert werden können. Das Material für das Analyseelement-Teilmagazin muss eine sehr gute Wasserdampfbarriere bilden, damit die Analyseelemente trocken gehalten werden. Bei der Verwendung von photometrischen Analyseelementen muss das Material des Teilmagazins wenigstens in Teilbereichen, bevorzugt vollständig, durchsichtig sein. Bevorzugt ist das Analyseelement-Teilmagazin aus einem transparenten, durchsichtigen Kunststoff hergestellt, jedenfalls der transparente Bereich. Geeignet sind polymere Werkstoffe, vorzugsweise ein Cycloolefin-Copolymer, also ein amorphes, transpatentes Copolymer auf Basis von zyklischen und linearen Olefinen, die sich auch durch eine hohe Transparenz, gute Feuchtigkeitsbarriere und hohe Steifigkeit mit geringem Verzug auszeichnen.

Bei dem Material des Stechelement-Teilmagazins kommt es hingegen darauf an, dass es keine Additive enthält, die das Stechelement und deren in der Regel hydrophile Schicht beeinträchtigen. Das Material darf auch nicht durch die zur Sterilisation der Stechelemente verwendete (Beta)-Strahlung beschädigt werden.

Bevorzugt bestehen beide Teilmagazine aus Kunststoffmaterialien und sind vorzugsweise im Spritzgussverfahren hergestellt. Die Kunststoffe sind entsprechend den Anforderungen ausgewählt.

Im Rahmen der Erfindung wurde festgestellt, dass die Verwendung integrierter Probengewinnungs- und Analyseelemente, wie sie in den zitierten Dokumenten (1) bis (3) beschrieben sind, erhebliche Nachteile hat. Die Integration führt zu erhöhten Herstellungskosten. Außerdem besteht das Risiko der Kontamination der Stechspitze des Probengewinnungsteils durch Reagenzien des Analyseteils. Ein erfindungsgemäßes zweiteiliges Magazin ermöglicht es, die Vorteile getrennter Stech- und Analyseelemente zu nutzen, erlaubt aber gleichzeitig eine kompakte Bauweise und einfache mechanische Konstruktion.

Bevorzugt ist das erfindungsgemäße Magazin ein Trommelmagazin, das eine Stechelement-Teiltrommel und eine Analyseelement-Teiltrommel aufweist. Die Verwendung einer Magazintrommel ermöglicht insbesondere eine sehr kompakte Bauweise. Hinsichtlich der Systemgröße, bezogen auf die Anzahl der möglichen Tests zur Bestimmung des Analyten, erscheinen Magazintrommeln nach dem gegenwärtigen Kenntnisstand das wohl erfolgsversprechendste und attraktivste Konzept. Alternativ kann das Magazin ein (quaderförmiges) Linearmagazin sein.

In einer bevorzugten Ausführung des Magazins mit zwei Teilmagazinen sind drei Sperrfolien vorhanden, nämlich eine erste, durch die ein Verbindungselement an das Stechelement ankoppeln kann, eine zweite, die zwischen den beiden Teilmagazinen angeordnet ist und die Stechelementkammer von der Analyseelementkammer trennt, und eine dritte, durch die das Stechelement aus dem Magazin auf seinem (geraden) Bewegungsweg in Einstichrichtung heraustreten kann. Bevorzugt weist das Magazin vier Sperrfolien auf, so dass jede Stirnfläche der Teilmagazine mit einer Folie verschlossen ist.

Die Erfindung wird nachfolgend anhand von in den Figuren dargestellten bevorzugten Ausführungsformen näher erläutert. Die darin dargestellten Besonderheiten können einzeln oder in Kombination verwendet werden, um bevorzugte Ausgestaltungen der Erfindung zu schaffen. Die beschriebenen Ausführungsformen stellen keine Einschränkung der Allgemeinheit des in den Ansprüchen definierten Gegenstandes dar. Es zeigen:
- Fig. 1: ein Prinzipschaltbild eines Analysesystems umfassend ein Analysegerät und ein Magazin;
- Fig. 2: eine Detailzeichnung einer ersten Ausführungsform eines Magazins;
- Fig. 3: eine weitere Ansicht des Magazins aus Figur 2;
- Fig. 4: eine Detailzeichnung eines Stechelementes;
- Fig. 5: eine weitere Ausführungsform eines Magazins mit einem Andruckelement;
- Fig. 6: eine Detailansicht des Magazins aus Figur 6;
- Fig. 7a,b,c: Detailansicht eines Andruckelements aus Figur 5;
- Fig. 8: eine weitere Ausführungsform eines Magazins;
- Fig. 9: eine weitere Ausführungsform eines Magazins.

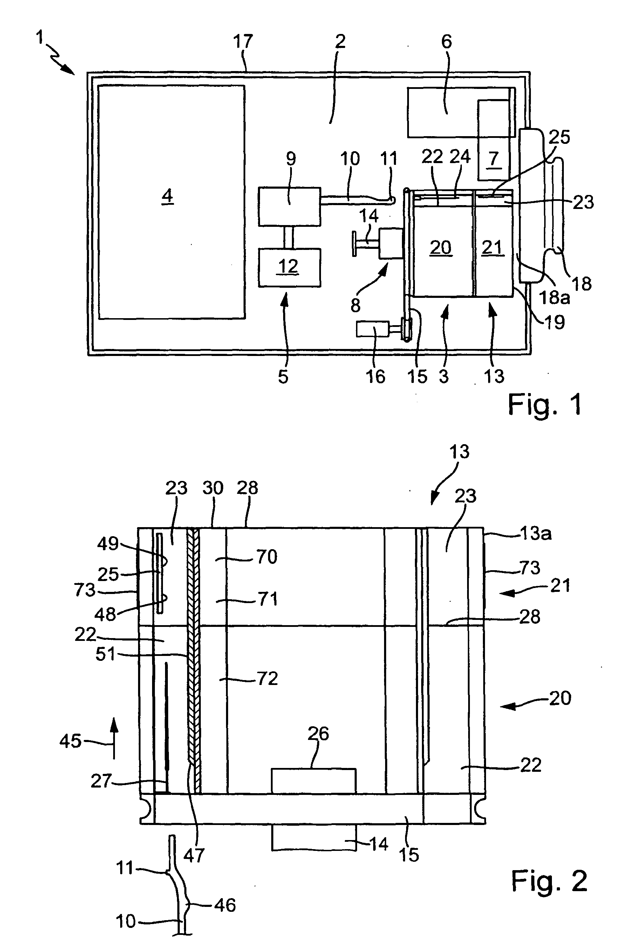

Ein erfindungsgemäßes Analysesystem 1 umfassend ein Analysegerät 2 und ein Magazin 3 ist in Figur 1 dargestellt.

Das Analysegerät 2 weist eine Stromversorgung 4, einen Stechantrieb 5, eine Mess- und Auswerteeinrichtung 6 mit einer optischen Messeinheit 7 und eine Halterung 8 zur Aufnahme des Magazins 3 auf. Der Stechantrieb 5 umfasst einen Kopplungsmechanismus 9 mit einem Verbindungselement 10, an dessen freien Ende sich ein Kupplungselement 11 befindet, und ein Antriebselement 12, dass ein elektrischer Motor ist. Die

Bewegung des Antriebselements 12 wird in eine Bewegung des Verbindungselements 10 und des mit dem Verbindungselement 10 gekoppelten Stechelements umgesetzt, so dass das Stechelement eine Stechbewegung in und entgegen der Einstichrichtung ausführt.

Das Magazin 3 ist bevorzugt als Trommelmagazin 13 ausgebildet, wie es auch in den nachfolgenden Figuren gezeigt ist. Die Halterung 8 zur Aufnahme des Magazins 3 umfasst eine Antriebswelle 14, die in eine hier nicht dargestellte Aufnahme des Magazins 3 eingreift, so dass die Bewegung der Welle 14 auf das Magazin 3 übertragen wird. Die Antriebswelle 14 wird über einen Riemenantrieb 15 von einem Motor 16 angetrieben.

Das Analysegerät 2 umfasst hier nicht dargestellte Komponenten, wie beispielsweise einen Prozessor, der die einzelnen Elemente steuert und den gewünschten Verfahrensablauf sicherstellt.

Das Analysegerät 2 hat ein Gehäuse 17 mit einem Hautkontaktring 18, an den eine Fingerbeere angelegt wird, um in der Haut eine Wunde zu erzeugen. Das Stechelement 3 tritt aus dem Magazin 3 (bevorzugt teilweise) heraus und dann durch den Hautkontaktring 18 aus dem Analysegerät 2 heraus und sticht in die Haut. Das Magazin 3 ist derart in der Halterung 8 gelagert, dass zwischen der in Einstichrichtung vorderen Stirnfläche 19 des Magazins 3 und dem Hautkontaktring 18 ein Abstand 18a vorhanden ist. Der Abstand 18a ist schon deshalb notwendig, weil der Durchstich einer die Stirnfläche 19 abdeckenden Folie zeitlich und räumlich getrennt von dem Einstich in den Finger erfolgen soll. Im Hinblick auf die gewünschte kompakte Bauweise des Analysegeräts 2 ist die axiale Länge dieses Zwischenraums (also der Abstand 18a) minimiert. Bevorzugt ist er höchstens 5 mm, sehr bevorzugt höchstens 2 mm und besonders bevorzugt höchstens 1 mm.

Das Trommelmagazin 13 ist ein zweiteiliges Magazin 3 mit einem Stechelement-Teilmagazin 20 und einem Analyseelement-Teilmagazin 21. Das Stechelement-Teilmagazin 21 hat einander benachbarte Stechelementkammern 22, in denen je ein Stechelement 24, das in axialer Richtung der Trommel ausgerichtet ist, enthalten ist. Das Analyseelement-Teilmagazin 21 umfasst einander benachbarte Analyseelementkammern 23, in denen je ein Analyseelement 25 enthalten ist. Die beiden Teilmagazine 20,21 des Magazins 3 sind derart ausgebildet, dass die Stechelementkammern 22 und die Analyseelementkammern 23 voneinander verschieden sind.

In Figur 1 ist das Magazin 3 in einer Funktionsposition in der Halterung 8 gelagert, in der das Verbindungselement 10 an ein Stechelement 24 in einer Stechelementkammer 22 ankoppeln kann. Nach einem erfolgten Einstich in die Haut und dem Wiederherausbewegen des Verbindungselements 10 aus der Stechelementkammer 22 wird das Magazin 3 um seine Rotationsachse weitergedreht, bis die nächste, bevorzugt benachbarte, Stechelementkammer 22 derart angeordnet ist, dass das Verbindungselement 10 an das in der Kammer befindliche Stechelement 24 ankoppeln kann. Das Magazin 3 ist dann wieder in Funktionsposition.

Das Magazin 3 kann in der Funktionsposition auch derart angeordnet sein, dass die optische Messeinheit 7 der Analyseelementkammer 23 benachbart ist. Bevorzugt werden das Magazin 3 bzw. die beiden Teilmagazine 20,21 so angeordnet, dass die Analyseelementkammer 23, der die Messeinheit 7 benachbart ist, mit der Stechelementkammer 22 fluchtet, in die das Verbindungselement 10 hineinbewegt werden kann. Eine photometrische Messung an photometrischen Analyseelementen 25 hat den Vorteil, dass ein sehr kleines, kostengünstiges Testfeld zur Messung verwendet werden kann und dass nur kleine Blutmengen für die Analyse notwendig sind. Außerdem ist die Trommel 13 einfach vor der photoelektrischen Messeinheit 7 zu plazieren.

Die photoelektrische Messeinheit 7 ist radial zu der Analyseelementkammer 23 derart angeordnet, dass ihre Optik senkrecht auf die Mantelfläche 13a des Trommelmagazins 13 ausgerichtet ist und eine Änderung der Messwerte im Analyseelement 25 photometrisch gemessen werden kann. Das Analyseelement-Teilmagazin 21 weist an seiner Mantelfläche 13a einen transparenten Bereich 73 auf, so dass die Messeinheit 7 durch den transparenten Bereich 73 die Änderung der Messgröße erfassen kann (bevorzugt wenn das Magazin 3 in der Funktionsposition ist). Das Analyseelement 25 ist zu dem transparenten Bereich 73 (z.B. ein transparentes Fenster) derart angeordnet, dass eine photometrische Messung erfolgen kann. Bevorzugt ist die gesamte Mantelfläche 13a transparent. Die Mantelfläche 13a des Trommelmagazins 13 weist ebene Abschnitte (Flächenabschnitt 76) derart auf, dass sie parallel zu den Analyseelementen 25 und der Optik der Messeinheit 7 sind. Die ebenen Flächenabschnitte 76 umfassen den transparenten Bereich 73 oder sind vollständig durchsichtig. Vorzugsweise hat auch das Stechelement-Teilmagazin 20 ebene Flächenabschnitte 76.

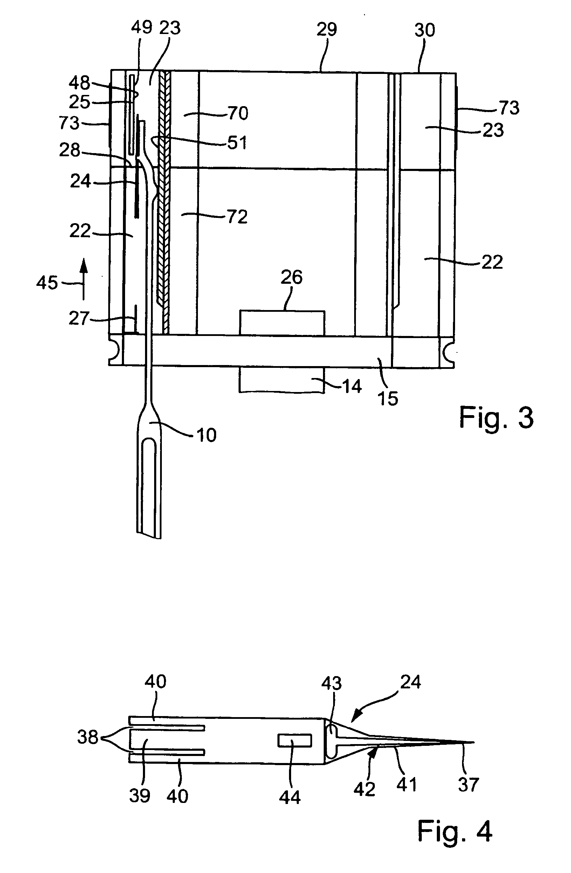

Die Figuren 2 und 3 zeigen das erfindungsgemäße Magazin 3 in seiner Funktionsposition, in die es mittels der Antriebswelle 14, die in eine Aufnahme 26 des Magazins 3 eingreift, gedreht wurde. Bevorzugt sind die Aufnahme 26 und das obere Ende der Antriebswelle 14 als Zahnkranz bzw. Zahnrad ausgebildet und bilden eine Zahnradverbindung, die ein zuverlässiges und genaues Positionieren des Magazins ermöglicht. In Figur 2 ist das Verbindungselement 10 des Stechantriebs 5 außerhalb des Magazins 3 angeordnet, in Figur 3 ist das Stechelement 24 auf seinem Bewegungsweg in einer Position etwa parallel zu dem Analyseelement 25 bewegt worden.

Die beiden Teilmagazine 20,21 des zylindrischen Trommelmagazins 13 sind in der Ausführungsform nach den Figuren 2,3 fest miteinander verbunden. Zwischen ihnen ist eine Verschlussfolie 28 angeordnet, die auf die jeweiligen Stirnseiten der Teilmagazine 20,21 aufgebracht bzw. aufgeklebt oder angeschweißt ist. Die Verschlussfolie 28 trennt die Stechelementkammern 22 und die Analyseelementkammern 23. Sie verschließt dabei eine Stechelementeintrittsöffnung 32 der Analyseelementkammer 23 und gleichzeitig eine Stechelementaustrittsöffnung 33 der Stechelementkammer 22.

Das Analyseelement-Teilmagazin 21 ist an seinem in Einstichrichtung vorderen Ende 29 bevorzugt mit einer Folie 30 derart abgedeckt, dass Magazinauslassöffnungen 31 der Analyseelementkammern 23 an der Vorderseite 19 verschlossen sind. Somit ergibt sich eine völlig geschlossene Analyseelementkammer 23. Die Verschlussfolien 28,30 der Analyseelementkammer 23 bilden eine Wasserdampfbarriere. Allen Folien ist gemein, dass sie nach dem Durchstoßen möglichst leicht weiterreißbar sind, um das Stechelement 24 auf seinem Bewegungsweg nicht zu behindern und während der Rückzugsphase der Stechbewegung das Stechelement 24 nicht berühren, um kein Blut von dem Stechelement 24 zu wischen.

Das Analyseelement 25 ist in der Analyseelementkammer 23 in einer hier nicht dargestellten Halterung gehalten. Das Analyseelement 25 ist vorzugsweise in einer in Axialrichtung (Einstichrichtung) festen Fluidtransferposition positioniert. Aufgrund ihrer Halterung und der geringen Dimensionen des Analyseelements 25 kann es mit Spiel gehalten sein. Jedenfalls ist seine Position in Einstichrichtung fest, so dass ein Fluidtransfer von dem Stechelement 24 auf das Analyseelement 25 möglich ist.

In einem bevorzugten Ausführungsbeispiel ist die Stechelementkammer 22 an dem in Einstichrichtung hinteren Ende 35 des Magazins 3 ebenfalls mit einer Verschlussfolie 36 abgedeckt, so dass eine Verbindungselementeintrittsöffnung 34 der Stechelementkammer 22 verschlossen ist. Die Verschlussfolie 36 wird - vorzugsweise mittels des Verbindungselements 10 - vor der Stechbewegung geöffnet, um den Eintritt des Verbindungselements 10 in die Stechelementkammer 22 zu ermöglichen. Durch die beiden Verschlussfolien 28,36 ist die Stechelementkammer 22 hermetisch geschlossen. Dadurch wird sichergestellt, dass die Sterilität des Stechelements 24 während der Lagerung erhalten bleibt. Die Folien 28,36 dürfen deshalb durch den Sterilisierungsprozess nicht beschädigt werden.

Das Stechelement 24, das in Figur 4 im Detail dargestellt ist, ist in einer Stechelementhalterung 27 der Stechelementkammer 22 eingeklemmt. Das vorzugsweise flach ausgebildete Stechelement 24 hat eine Spitze 37 und an seinem der Spitze 37 gegenüberliegenden Ende zwei Schlitze 38 derart, dass eine mittlere Zunge 39 und zwei Randzungen 40 gebildet werden. Die Zungen 39,40 können elastisch verbogen werden, damit das Stechelement 24 auf die vorzugsweise als angespitzte Nase ausgebildete Stechelementhalterung 27 aufgesetzt werden kann. Das Stechelement 24 ist vor und nach Durchführung der Stechbewegung in der Halteposition in der Stechelementhalterung 27 angeordnet und sicher gelagert. Das bevorzugt flache Stechelement 24 kann durch Ätzung hergestellt werden und ist entsprechend preiswert. Aufwendigen Biegevorgänge zur Formgebung sind nicht notwendig.

Von der Spitze 37 des Stechelements 24 erstreckt sich an einer Flachseite ein Kapillarkanal 41 bis zu einer Probenübergabezone 42. Der Kapillarkanal 41 kann auch beidseitig offen sein, z.B. kann er als (durchgängiger) Schlitz ausgebildet sein. Die Probenübergabezone 42 ist ein räumlicher Bereich innerhalb des Kapillarkanals 41, der in der Übergabeposition mit einer Probenkontaktzone eines Analyseelements 25 in Kontakt kommen kann. In einer bevorzugten Ausführungsform erstreckt sich der Kapillarkanal 41 über die Probenübergabezone 42 hinaus in eine Probenüberschusszone 43 ("waste zone"). Diese bevorzugte Ausführungsform des Stechelements 24 weist den Vorteil auf, dass eine Übertragung einer Körperflüssigkeit bevorzugt erst dann stattfindet, wenn eine erste Teilmenge der Körperflüssigkeit, die zuerst nach dem Einstich in die Haut in dem Kapillarkanal 41 aufgenommen wird, die Probenübergabezone 42 passiert hat und sich in der Probenüberschusszone 43 befindet. Eine zweite Teilmenge der Körperflüssigkeitsprobe befindet sich (zu dem Zeitpunkt) in der Probenübergabezone 42, so dass die zweite Teilmenge auf eine Probenkontaktzone eines Analyseelements 25 übertragen wird. Ein zeitlich gesteuerter Probenübertrag wird so möglich.

Das Stechelement 24 weist eine als Kupplungsaufnahme 44 ausgebildete Kupplungsstruktur auf, die zum Beispiel als Öffnung ausgebildet ist. Das korrespondierende Kupplungselement 11 des Verbindungselements 10 greift in die Kupplungsaufnahme 44 ein, um eine formschlüssige Verbindung herzustellen, damit das Stechelement 24 auf seinem Bewegungsweg bewegt werden kann. Hierdurch wird eine vorzugsweise bidirektional wirkende Kopplung des Stechelements 24 mit dem Stechantrieb 5 bewirkt.

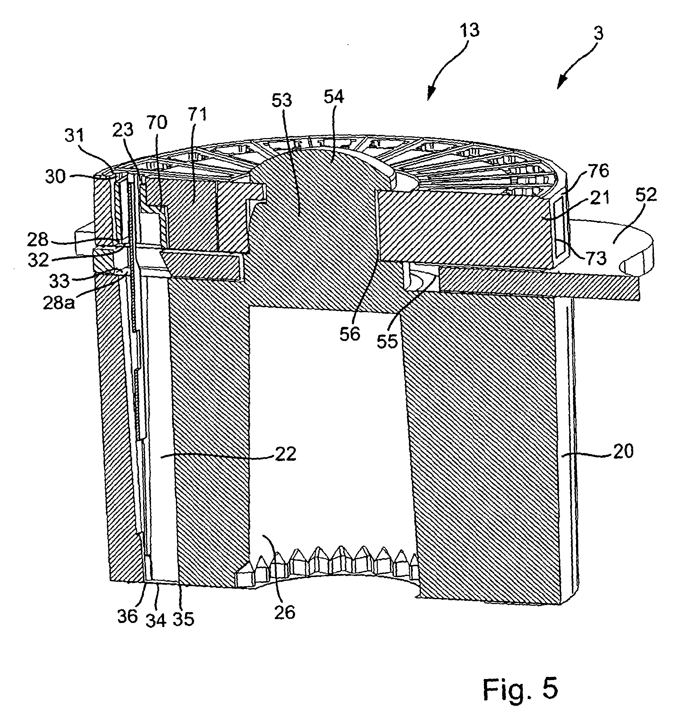

Der prinzipielle Bewegungsablauf wird anhand der Figuren 2 und 3 beschrieben. Für andere Ausführungsformen spezifische Bewegungen werden anhand der Figuren 5, 6 und 8 erläutert.

Ausgehend von Figur 2 wird das Verbindungselement 10 in Einstichrichtung 45 (Pfeilrichtung) bewegt. Das beispielsweise als Schubstange ausgebildete Verbindungselement 10 wird vom hinteren Ende 35 an das Magazin 3 herangeführt und durchstößt die Folie 36, um durch die Verbindungselementeintrittsöffnung 34 in die Stechelementkammer 22 einzudringen. Dabei wird während der weiteren Bewegung des Verbindungselements 10 ein Führungsnocken 46 des Verbindungselements 10 an einer Rampe 47 in der Stechelementkammer 22 derart geführt, dass das Kupplungselement 11 in die Kupplungsaufnahme 44 des Stechelements 24 eingreift und das Verbindungselement 10 an das Stechelement 24 (in seiner Halterung) ankoppelt.

Auf dem weiteren wenigstens teilweise gradlinigen Bewegungsweg in Einstichrichtung 45 durchsticht das Stechelement 24 die Verschlussfolie 28 zwischen den Teilmagazinen 20,21, während es durch die Stechelementaustrittsöffnung 33 und die Stechelementeintrittsöffnung 32 in die Analyseelementkammer 23 bewegt wird, bis es schließlich durch die Magazinauslassöffnung 31 aus dem Magazin 3 teilweise heraustritt und dabei die Folie 30 durchstößt. Diese bevorzugt größtenteils gradlinige Vortriebsphase der Stechbewegung endet im Umkehrpunkt, an dem der Einstich in die Haut erfolgt. An dem Umkehrpunkt schließt sich eine Rückführungsphase entgegen der Einstichrichtung 45 an. Wenigstens ein Teil der Vortriebsphase der Stechbewegung wird als schnelle Stechbewegung durchgeführt, wobei bevorzugt das Durchstechen der Verschlussfolie 28 zwischen den beiden Teilmagazinen 20,21 langsam stattfindet. Es kann vorgesehen sein, dass zwischen einer langsamen Phase und einer schnellen Phase der Vortriebsphase das Stechelement angehalten wird, dies ist jedoch nicht zwangsläufig.

Das Magazin 3 ist derart ausgebildet, dass das Stechelement 24 während der gesamten Stechbewegung in dem Magazin 3 geführt wird. Vorzugsweise wird die Führung durch das Stechelement-Teilmagazin 20 bewirkt. Ein mit seiner Spitze 37 aus dem Magazin 3 austretendes und eine Wunde erzeugendes Stechelement 24 ist mit seinem hinteren Ende immer noch in der Stechelementkammer 22 angeordnet. Dabei erstreckt sich das Stechelement 24 durch die gesamte Analyseelementkammer 23, deren Länge folglich deutlich kleiner ist als die Länge der Stechelementkammer 22.

Die Führung des Stechelements 24 auf seinem Bewegungsweg wird von der Kammerseitenwand 51 (Wandung) der Stechelementkammer 22 definiert, beispielsweise in dem sich das Verbindungselement 10 (beispielsweise mit seinem Führungsnocken 46) an der Wandung der Kammer 22 abstützt.

Nachdem das Stechelement 24 aus dem Magazin 3 herausgetreten ist und in die an dem Hautkontaktring 18 anliegende Haut einer Fingerkuppe eingestochen hat, wird das Stechelement 24 (während der Rückführungsphase) in eine Übergabeposition innerhalb der Analyselelementkammer 23 bewegt, Figur 3.

Nach dem Erzeugen der Einstichwunde wird Körperflüssigkeit (Blut) aus der Wunde in den Kapillarkanal 41 aufgenommen und durch Kapillarkräfte zur Probenübergabezone 42 des - bevorzugt hydrophilisierten - Stechelements 24 weitergeleitet. In der Übergabeposition hat eine erste Teilmenge der Körperflüssigkeit die Probenübergabezone 42 bereits passiert und eine zweite Teilmenge ist in die Probenübergabezone 42 gelangt. Da die erste Teilmenge beispielsweise durch Schweiss verunreinigt sein kann, wird sie nicht zur Analyse verwendet.

Das in der Fluidtransferposition in axialer Richtung positionierte Analyseelement 25 weist eine Probenkontaktzone 48 auf, die bevorzugt eine in Einstichrichtung ausgerichtete Fläche 49 einschließt, die sich in etwa parallel zur Einstichrichtung 45 erstreckt. Der Fluidkontakt zwischen dem Stechelement 24 und dem Analyseelement 25 wird bevorzugt durch Kontaktierung der Fläche 49 der Probenkontaktzone 48 mit der Probenübergabezone 42 des Stechelements 24 hergestellt.

Um den Abstand 50 zwischen der Probenkontaktzone 48 und der Probenübergabezone 42 zu überwinden, wird bevorzugt eine Relativbewegung zwischen Analyseelement 25 und Stechelement 24 durchgeführt. Bevorzugterweise ist der Abstand 50 höchstens 1 mm, vorzugsweise höchstens 0,5 mm und besonders bevorzugt höchstens 0,3 mm. Diese Relativbewegung kann beispielsweise eine Querbewegung sein. Eine Möglichkeit zur Ausführung der Querbewegung ist ein Verschieben oder Verschwenken des Verbindungselements 10.

Nach der Übertragung der Körperflüssigkeit auf die Probenkontaktzone 48 findet eine photometrische Messung (z.B. Reflexionsphotometrie, Absorptions- oder Fluoreszenzmessung) statt. Alternativ kann eine elektrochemische Messung (z.B. Amperometrie, Potentiometrie) erfolgen.

Anschließend wird das Stechelement 24 in seine Halteposition zurückbewegt und in der Stechelementhalterung 27 positioniert. Das Verbindungselement 10 wird vom Stechelement 24 entkoppelt und aus dem Magazin 3 heraus bewegt.

Bevorzugt wird die Relativbewegung zwischen dem Stechelement 24 und dem Analyseelement 25 dadurch bewirkt, dass ein Andruckelement quer zu der Einstichrichtung gegen das in der Übergabeposition befindliche Stechelement 24 gedrückt wird. Eine mögliche Ausführungsform eines derartigen Andruckelements ist in den nachfolgenden Figuren 5 bis 7 beschrieben. Alternativ könnte ein Andruckelement außerhalb des Analyseelement-Teilmagazins 21 an dessen vorderen Ende 29 ein herausragendes Stechelement 24 quer zur Einstichrichtung bewegen, um einen Kontakt mit dem Analyseelement 25 zu bewirken.

Die Figuren 5 bis 7 zeigen eine alternative Ausführungsform eines erfindungsgemäßen Magazins 3, das ebenfalls ein Trommelmagazin 13 ist. Das Stechelement-Teilmagazin 20 und das Analyseelement-Teilmagazin 21 sind derart voneinander beabstandet, dass zwischen ihnen ein Andruckelement 52 positioniert ist.

Das Stechelement-Teilmagazin 20 weist an seinem hinteren Ende 35 eine teilweise zahnkreisartig ausgebildete Aufnahme 26 für die hier nicht dargestellte Antriebswelle 14 auf. In Verlängerung der Aufnahme 26 hat das Stechelement-Teilmagazin 20 einen Dorn 53 mit einem (überstehenden) Hülsenkopf 54. Der Dorn 53 erstreckt sich durch eine Durchgangsöffnung 55 des Andruckelements 52 und eine Öffnung 56 des Analyseelement-Teilmagazins 21. Der gegenüber der Öffnung 56 verbreiterte Hülsenkopf 54 ragt aus dem Magazin 3 heraus und verbindet die beiden Teilmagazine 20,21 und das Andruckelement 52 derart, dass sie nicht voneinander gelöst werden können. Vorzugsweise korrespondieren der Dorn 53 und die Öffnung 56, um eine ortsfeste (rotationsfeste und radial- und axialfixierte) Verbindung zwischen den Teilmagazinen 20,21 zu erzeugen. Eine Relativbewegung zwischen den beiden Teilmagazinen 20,21 ist ausgeschlossen.

Im Gegensatz zur Ausführungsform nach den Figuren 2 und 3 sind die Teilmagazine 20,21 derart angeordnet, dass die Stechelementeintrittsöffnung 32 und die Stechelementaustrittsöffnung 33 voneinander beabstandet sind. Beide Öffnungen 32,33 sind deshalb mit separaten Verschlussfolien 28,28a verschlossen. Die Folie 28a wird bevorzugt vor oder während der Stechbewegung des Stechelements durchstoßen, bevorzugt von dem Stechelement 24 selbst.

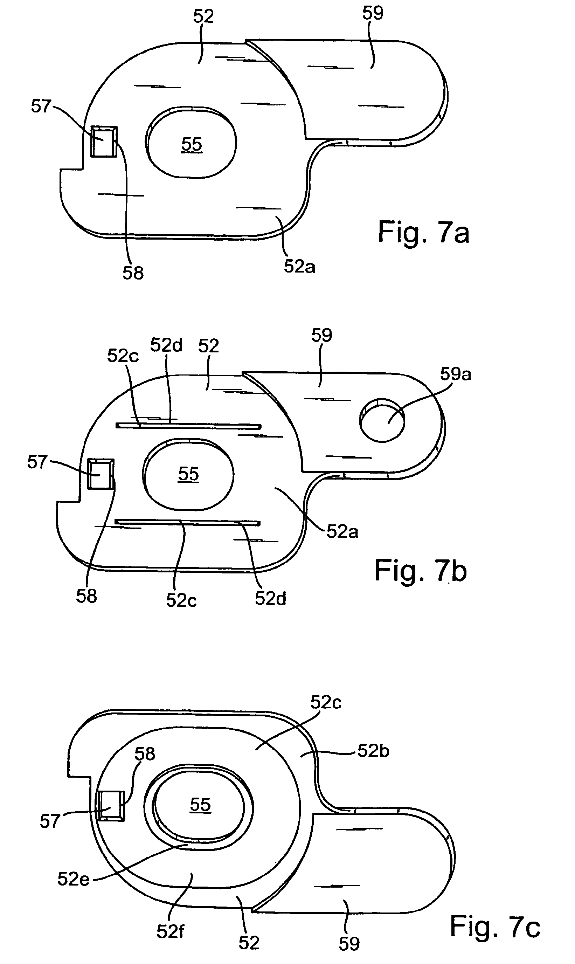

Die Durchgangsöffnung 55 des Andruckelementes 52 ist bevorzugt ein Langloch, wie in den Figuren 7a-c dargestellt. Das Langloch ermöglicht eine Bewegung des Andruckelements 52 in radiale Richtung zwischen den beiden Teilmagazinen 20,21. Es wird bevorzugt von dem Analysegerät 2 bewegt (und geführt), wobei ein nicht dargestelltes Bewegungselement vorzugsweise an den zungenartigen Ansatz 59 angreift. Die Rotationsbewegung des Trommelmagazins 13 und die translatorische Bewegung des Andruckelements 52 in Radialrichtung (quer zur Einstichrichtung) sind vollständig entkoppelt. Das plattenförmige Andruckelement 52 hat eine Stechelementführungsöffnung 57 mit einer vorzugsweise abgeschrägten Andruckfläche 58, die durch eine Seitenwand der Stechelementführungsöffnung 57 gebildet wird.

In einer Ausführungsform des plattenförmigen Andruckelements 52 nach Figur 7b sind an seiner Oberseite 52a Führungsstrukturen 52c angeordnet, die das Analyseelement-Teilmagazin 21 kontaktieren. Die Führungsstruktur 52c ist als Führungsschiene 52d ausgebildet. Das Andruckelement 52 berührt das Teilmagazin 21 nur an den Rippen 52d, so dass die Reibung während der Bewegung des Andruckelements 52 deutlich reduziert wird. Gleichzeitig wird ein definierter Abstand zwischen der Oberseite 52a und dem Analyseelement-Teilmagazin 21 erreicht. Eventuell abstehende Folienteile der Verschlussfolie des Analyseelement-Teilmagazins 21 können sich in den Zwischenraum (Spalt) erstrecken ohne die Relativbewegung zwischen Andruckelement 52 und Teilmagazin 21 zu behindern. Bevorzugt ist an der Unterseite 52b ebenfalls eine Führungsstruktur 52c ausgebildet, z.B. als Führungsschiene 52d oder Rippe, wodurch die Reibung weiter reduziert und das Andruckelement 52 in einer definierten Position zwischen den beiden Teilmagazinen 20,21 gelagert wird.

In einer alternativen Ausführungsform nach Figur 7c ist die Führungsstruktur 52c an der Unterseite 52b des Andruckelements 52 als Aufnahmeraum 52f ausgebildet. Der Aufnahmeraum 52f erstreckt sich um den Rand der Durchgangsöffnung 55 herum. Zwischen dem Aufnahmeraum 52f und der Durchgangsöffnung 55 wird ein gegenüber der Vertiefung des Aufnahmeraums 52f erhöhter Rand gebildet, der beispielsweise ein Wulst 52g sein kann. Die Reibung beim Drehen der Magazintrommel 13 relativ zu dem Andruckelement 52 wird reduziert. Von der geöffneten Verschlussfolie 28a der Stechelementaustrittsöffnung 33 hervorstehende Folienteile können sich in den vertieften Aufnahmeraum 52f erstrecken, ohne die Bewegung zwischen dem Andruckelement 52 und den TeilMagazinen 20,21 zu beeinflussen. Hierdurch kann die für das Drehen des Magazins 3 in der Halterung 10 aufgewandte Kraft reduziert werden, was den Energieverbrauch minimiert, da das Andruckelement 52 in Bezug auf das Analysegerät 2 rotationsfest positioniert ist (in Bezug auf das Analysegerät 2 ist nur eine translatorische Linearbewegung des Andruckelements 52 möglich).

Der zungenartige Ansatz 59 kann eine Griffmulde 59a aufweisen, die dem Benutzer eine Griffmöglichkeit beim Einsetzen des Magazins 3 in die Halterung 10 des Analyseelements 2 bietet. Ein Berühren der transparenten Bereiche oder der Mantelfläche wird verhindert, was später ein optisches Messergebnis verfälschen könnte.

In einer weiter bevorzugten Ausführungsform weist das Magazin 3, bevorzugt das Andruckelement 52, eine Codierung auf, mittels der Informationen über eine Auswertekurve oder Auswertefunktion zum Umrechnen der Messwerte in die gesuchten analytischen Resultate codiert sein können. Da die Auswertefunktion oder Auswertekurve in der Regel produktionschargenspezifisch für die einzelnen Analyseelemente ist, können die zur Auswertung benötigten Informationen in der Codierung enthalten sein. Die Codierung kann in Form eines Codes, beispielsweise eines Barcodes (2D-Barcode) oder in Form eines Transponders, beispielsweise eines RFID-Transponders (Radio Frequency Identification-Transponder) realisiert sein.

In der Stechposition, in der sich das Trommelmagazin 13 zu Beginn des Stechvorgangs befindet, sind das Stechelement-Teilmagazin 20 und das Analyseelement-Teilmagazin 21 derart ausgerichtet, dass eine Stechelementkammer 22, eine Analyseelementkammer 23 und die Stechelementführungsöffnung 57 fluchten. Das Stechelement 24 kann ungehindert durch die Stechelementführungsöffnung 57 des Andruckelements 52 hindurch bewegt werden und führt die anhand der Figuren 2 und 3 beschriebene Stechbewegung durch. Sobald das Stechelement 24 in seiner Übergabeposition ist, wird eine Relativbewegung quer zur Einstichrichtung zwischen dem Stechelement 24 und dem Analyseelement 25 ausgeführt, um einen Fluidkontakt herzustellen.

Figur 5 zeigt das Stechelement 24 in einer Übergabeposition nach dem erfolgten Einstich. In dieser Position erstreckt sich das Stechelement 24 in der Analyseelementkammer 23 und in der Stechelementkammer 22 sowie durch die Stechelementführungsöffnung 57. Das Andruckelement 52 ist in seiner Ausgangsposition.

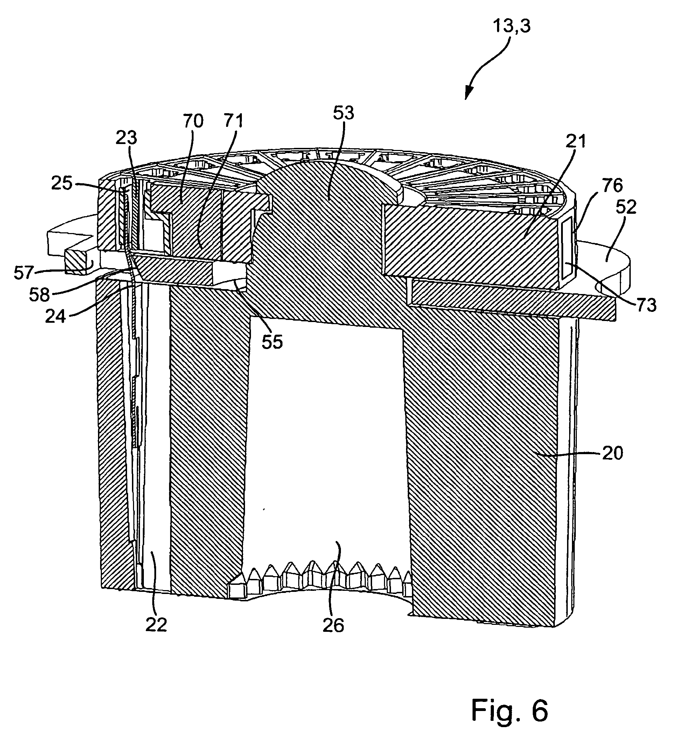

Figur 6 zeigt das Andruckelement 52 radial nach außen (von der Rotationsachse des Magazins weg in Richtung Messeinheit) verschoben. In dieser Andruckposition liegt die Andruckfläche 58 der Stechelementführungsöffnung 57 an dem Stechelement 24 an und verbiegt es elastisch derart, dass die Probenübergabezone 42 des Stechelements 24 mit der Probenkontaktzone 48 des Analyseelements 25 in Kontakt kommt und ein Fluidtransfer zwischen ihnen stattfindet.

Wenn die beiden Elemente 24,25 in Kontakt stehen, befindet sich das Analyseelement 25 vorzugsweise in einer Analyseposition, in der eine optische Erfassung einer Messgröße möglich ist. Wird das Analyseelement 25 in seiner Halterung mit Spiel in Querrichtung zur Einstichrichtung gehalten, so ist es jedenfalls beim Kontakt mit dem Stechelement 24 in eine definierte Analyseposition gelangt, die kein Spiel quer zur Einstichrichtung zulässt.

Nach der Analyse der Körperflüssigkeitsprobe wird das Andruckelement 52 radial in seine Ausgangsstellung zurückbewegt. Das (elastische) Stechelement 24 nimmt seine ursprüngliche Form wieder an. Der Bewegungsweg während der Rückführungsphase wird fortgesetzt.

In einer alternativen Ausführungsform des erfindungsgemäßen Magazins 3 mit einem Analyseelement-Teilmagazin 21 und einem Stechelement-Teilmagazin 20 sind die beiden Teilmagazine 20,21 relativ zueinander quer zur Einstichrichtung bewegbar.

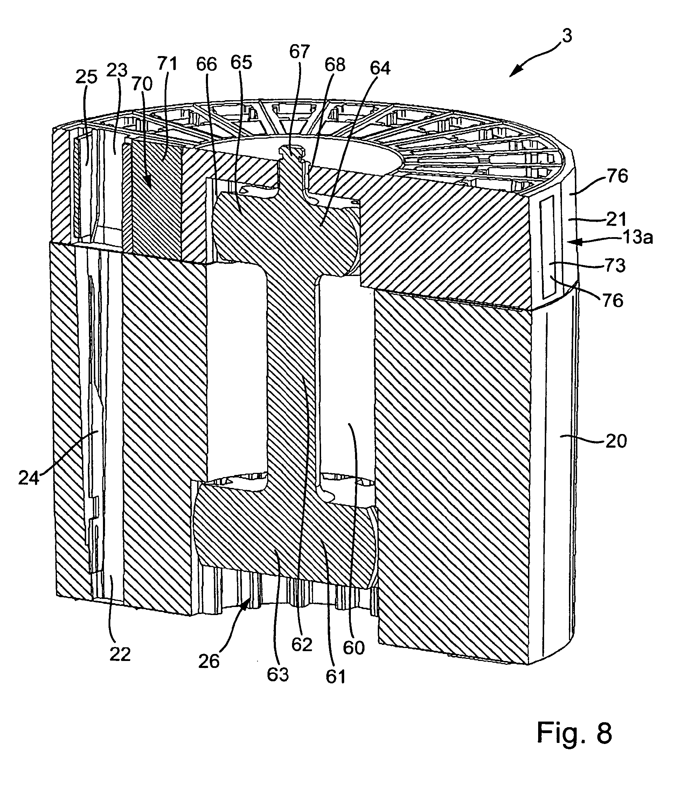

Eine besondere Ausführungsform eines solchen, als zweiteiliges Trommelmagazin 13 ausgebildeten, Magazins 3 wird anhand von Figur 8 beschrieben. Wie bei dem Ausführungsbeispiel der Figuren 5 und 6 sind auch bei diesem Magazin 3 die beiden Teilmagazine 20,21 an ihren jeweiligen Stirnseiten mit Folien 28,28a,30,36 abgedeckt, so dass die entsprechenden Öffnungen der Kammern 22,23 verschlossen sind.

In einer bevorzugten Ausführungsform der Erfindung werden die Folien 28,28a,30,36 zum Verschließen der Öffnungen 31,32,33,34 der Kammern 22,23 in einem Heißklebeverfahren mit einem Heißkleber mit den Stirnseiten der Teilmagazine 20,21 verklebt. Bevorzugt weisen die Öffnungen der Kammern 22,23 Heißkleberaufnahmeausnehmungen auf, in die überflüssiger Klebstoff (Heißkleber) aufgenommen werden kann. Die Heißkleberaufnahmeausnehmungen können beispielsweise als Vertiefungen ausgebildet sein, die den Rand der Öffnungen 31,32,33,34 umranden. Es können aber auch sich ins Magazininnere erstreckende Reservoirs vorgesehen sein.

Die beiden Teilmagazine 20,21 sind so angeordnet, dass je eine Stechelementkammer 22 mit einer Analyseelementkammer 23 fluchtet. Die beiden Teilmagazine 20,21 sind rotationsfest zueinander angeordnet. Mit anderen Worten: die beiden Teilmagazine 20,21 können nicht gegeneinander verdreht werden.

Das Stechelement-Teilmagazin 20 weist eine Durchgangsbohrung 60 auf, in der eine Aufnahme 26 integriert ist, um eine Antriebswelle 14 des Analysegeräts 2 aufzunehmen. In der zahnkranzförmigen Aufnahme 26 ist auch das untere Ende 61 einer Kardanwellen-ähnlichen Gelenkwelle 62 positioniert. Ein unterer Gelenkwellenkopf 63 der Gelenkwelle 62 ist als Zahnrad ausgebildet und greift in die zahnkranzförmige Aufnahme 26 ein. Am oberen Ende 64 der Gelenkwelle 62 ist ein oberer zahnradförmiger Gelenkwellenkopf 65 angeordnet, der in eine sackbohrungsähnliche Gelenkwellenaufnahme 66 des Analyseelement-Teilmagazins 21 eingreift.

Oberhalb des Gelenkwellenkopfes 65 ist ein stiftartiger Fortsatz 67 angeformt, der durch eine Stirnwand-Öffnung 68 in der Stirnwand des Analyseelement-Teilmagazins 21 ragt. In einer außerhalb des Magazins 3 angeordneten Nut des Fortsatzes 67 kann ein Splint eingeclipst werden, so dass die Gelenkwelle 62 nicht aus dem Magazin 3 entfernt werden kann. Die beiden Teilmagazine 20,21 sind über die Gelenkwelle 62 rotationsfest miteinander verbunden und zusätzlich axial fixiert, d.h. eine Relativbewegung der Teilmagazine 20,21 in axialer Richtung ist ausgeschlossen.

Das Analyseelement-Teilmagazin 21 und das Stechelement-Teilmagazin 20 sind bevorzugt relativ zueinander quer zur Einstichrichtung bewegbar, insbesondere radial beweglich. Die Gelenkwelle 62 lässt sich in der Gelenkwellenaufnahme 66 derart bewegen, dass eine Radialverschiebung quer zur Einstichrichtung möglich ist. Durch die Relativbewegung der beiden Teilmagazine 20,21 zueinander, kann die Position des Analyseelements 25 in radialer Richtung derart verändert werden, dass ein Kontakt zwischen dem Analyseelement 25 und dem in der Übergabeposition angeordneten Stechelement 24 bewirkt wird. Die Relativbewegung umfasst einen Querbewegungsweg (Versatz) von bevorzugt wenigstens 0,1 mm, sehr bevorzugt mindestens 0,3 mm, besonders bevorzugt von mindestens 0,5 mm. Der Versatz kann auch bis zu 1,5 mm sein. Die Relativbewegung kann beispielsweise durch einen an dem Fortsatz 67 angreifenden Bewegungsmechanismus oder durch einen seitlich an ein Teilmagazin 20,21 andrückenden Stößel oder einen ähnlichen Mechanismus bewirkt werden. Bevorzugt wird das Analyseelement-Teilmagazin 21 bewegt. In dieser radial verschobenen (ausgelenkten) Position erfolgt bevorzugt eine optische Auswertung des Analyseelementes 25.

Nach der Übertragung der Körperflüssigkeit wird das Analyseelement-Teilmagazin 21 in seine Ausgangslage zurückbewegt, so dass die Analyseelementkammer 23 wieder mit der Stechelementkammer 22 fluchtet. Die optische Auswertung des Analyseelements 25 kann alternativ auch in dieser Ausgangslage durchgeführt werden.

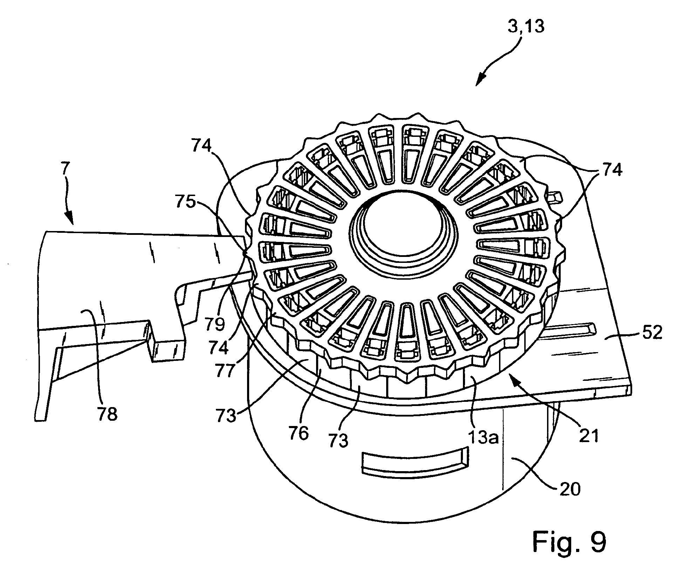

Figur 9 zeigt eine weitere Besonderheit eines Magazins 3. In der Mantelfläche 13a des Analyseelement-Teilmagazins 21 sind Positionierelemente 74 angeordnet, die das Positionieren der photoelektrischen Messeinheit 7 ermöglichen. Die Positionierelemente 74 können beispielsweise in Form von Zähnen 77 ausgebildet sein. Die Messeinheit weist einen (beweglichen) Positionierarm 78 mit einer Positionierkontur 75 an dessen Ende auf, die mit dem Positionierelement 74 des Magazins 3 korrespondiert.

Die als Positionierelementaufnahme 79 ausgebildete Positionierkontur 75 bildet die negative Form eines Zahns 77, so dass das Positionierelement 74 in der Positionierkontur 75 aufgenommen werden kann.

Die Positionierelemente 74 sind, wie an dem vorderen Ende 29 des Analyseelement-Teilmagazins 21 angeordnet, so dass sich ein umlaufender Zahnkranz bildet. Sie sind bevorzugt soweit am vorderen Ende 29 positioniert, dass der transparente Bereich 73 der Mantelfläche 13a frei bleibt. Die Positionierelemente 74 sind an das Analyseelement-Teilmagazin 21 angespritzt.

Durch das Zusammenwirken von Positionierelement 74 und Positionierkontur 75 wird ein reproduzierbarer gleicher Messabstand zwischen dem Analyseelement 25 und der Optik der Messeinheit 7 gewährleistet. Gleichzeitig wird durch die zahnkranzartige Ausbildung der Positionierelemente 74 auch eine Rotationspositionsfixierung vorgenommen. Die senkrecht zur Rotationsachse (radial) bewegbare Messeinheit 7 ist in Tangentialrichtung des Magazins 3 positioniert. Durch eine geeignete Ausbildung von Positionierkontur 75 und Positionierelement 74 wird eine axiale Positionierung von Messeinheit 7 und Analyseelement-Teilmagazin 21 zueinander erreicht.

Die Führung der Messeinheit 7 und deren exakter Positionierung (Abstand und axiale Position) zu dem Teilmagazin 21, insbesondere deren Kontaktverbindung, gewährleistet eine exakte und fehlerfreie Messung. Um die Messung nicht durch ein Bewegen der Messeinheit 7 relativ zu dem Magazin 3 zu verfälschen, wird der Kontakt zwischen Messeinheit 7 und Magazin 3 vor der Kontaktierung des Stechelements 24 mit dem Analyseelement 25, bevorzugt vor Ausführung des Stechvorgangs, besonders bevorzugt schon in der Funktionsposition des Magazins 3, hergestellt, also bevor das Verbindungselement 10 des Analysegeräts 2 mit dem Stechelement 24 ankoppelt.

Durch den Kontakt zwischen Messeinheit 7 und Magazin 3 werden die maßliche Toleranzen der einzelnen Elemente und ein mögliches Spiel zwischen dem Magazin 3 und der Halterung 10 des Analyseelements 2 teilweise kompensiert, jedenfalls jedoch minimiert.

Die Verwendung von Positionierelementen 74 kann selbstverständlich an allen hier beschriebenen Magazinen 3 vorgesehen sein. Sie eignet sich immer bei einer photometrischen Messung der Körperflüssigkeit, da der Messabstand exakt eingestellt werden kann.

In einer ebenfalls bevorzugten Ausführungsform sind an der Mantelfläche 13a des Stechelement-Teilmagazins 20 Ausrichtungselemente angeordnet, die beim Herstellungsprozess des Magazins 3, wenn die beiden Teilmagazine 20,21 zusammengefügt werden, eine Relativpositionierung der beiden Teilmagazine 20,21 zueinander erleichtern. Mit Hilfe der Ausrichtungselemente und der Positionierelemente 74 kann eine einfache und genaue relative Ausrichtung der Kammern der beiden Teilmagazine 20,21 während der Montage erzielt werden.

Alle Ausführungsformen des Magazins 3 sind bevorzugt derart ausgebildet, dass zu je einer Analyseelementkammer 23 eine Trockenmittelkammer 70 benachbart angeordnet ist. Bevorzugt sind die Trockenmittelkammer 70 und die Analyseelementkammer 23 so miteinander verbunden, dass ein Luftaustausch zwischen den beiden Kammern 70,23 stattfinden kann. In der Trockenmittelkammer 70 ist ein Trockenmittel 71 enthalten, um Feuchtigkeit aufzunehmen und das Analyseelement 25 trocken zu halten.

In der Ausführungsform nach Figur 2 und 3 ist bevorzugt eine zweite Trockenmittelkammer 72 im Stechelement-Teilmagazin 20 enthalten. Die beiden fluchtenden Kammern 70,72 bilden eine Trockenmittel-Gesamtkammer. Wenn die Kammern 70,72 durch eine Verschlussfolie 28 voneinander getrennt sind, wird diese im Herstellungsprozess durchstoßen. Nur die Trockenmittelkammer 70 steht direkt mit der Analyseelementkammer 23 so in Verbindung, dass ein Luftaustausch stattfinden kann. Ein Austausch zwischen einer Trockenmittelkammer 70,72 und der Stechelementkammer 22 ist nicht möglich. Diese Ausführungsform hat den Vorteil, dass mehr Trockenmittel zur Verfügung steht, um das Analyseelement 25 in der Analyseelementkammer 23 trocken zu halten.

Den gezeigten Ausführungsformen des erfindungsgemäßen Magazins 3 ist gemeinsam, dass der Ablauf der Stechbewegung durch einen Bewegungsmechanismus, der den Stechantrieb 5 einschließt, bewirkt wird. Der Bewegungsmechanismus ist so ausgebildet, dass die einzelnen Schritte, insbesondere die Bewegungsphasen des Stechelements automatisch stattfinden. Ein Eingriff des Benutzers ist nicht erforderlich. Vielmehr betätigt der Benutzer das Analysegerät 2 nur zu Beginn des Probengewinnungs- und Analysevorgangs, nachdem er seinen Finger an den Hautkontaktring 18 angelegt hat. Nach Durchführung der Stechbewegung und der Analyse kann der Benutzer das analytische Resultat oder eine damit korrespondierende Größe auf beispielsweise einem Display ablesen. Alle notwendigen Zwischenschritte werden von dem Analysegerät automatisch und selbsttätig durchgeführt. Dazu gehört auch das Bewegen des Magazins 3 in eine Funktionsposition und das Weitertakten des Magazins 3 derart, dass es nach Durchführung eines Einstichs und einer Analyse in eine neue Funktionsposition bewegt wird, in der das Verbindungselement 10 mit einem neuen, noch unbenutzten Stechelement 24 in Kontakt kommen und das Stechelement 24 auf seinem Einstichweg bewegen kann.

## Patentansprüche

1. Analysesystem zur Bestimmung eines Analyten in einer durch einen Stich in die Haut gewonnenen Körperflüssigkeitsprobe, umfassend
ein Magazin (3) mit Kammern, enthaltend
- Analyseelemente (25) mit einer Probenkontaktzone (48) und einem mindestens ein Reagenz enthaltenden Reagenzsystem, dessen Reaktion mit der Körperflüssigkeit zu einer messbaren Änderung einer Messgröße führt,
- Stechelemente (24) mit einer Spitze (37) zum Einstechen in die Haut mit einem Kapillarkanal (41), der eine Fluidverbindung zwischen der Spitze (37) und einer Probenübergabezone (42) des Stechelementes (24) bildet,
ein wiederverwendbares Analysegerät (2) mit
- einem Stechantrieb (5) zum Antreiben einer Stechbewegung eines Stechelementes (24) auf einem Bewegungsweg, der eine Vortriebsphase in Einstichrichtung und nach Erreichen eines Umkehrpunktes der Stechbewegung eine Rückführungsphase entgegen der Einstichrichtung umfasst,
- einer Alterung (8) zur Aufnahme des Magazins (3) derart, dass sich jeweils eine Kammer des Magazins (3) in einer Funktionsposition befindet, in der ein Stechelement (24) in der Kammer von dem Stechantrieb (5) bewegt werden kann, und
- einer Mess- und Auswerteeinrichtung (6) zur Messung der messbaren Änderung einer Messgröße und zur Ermittlung eines gewünschtes Analyseergebnisses, wobei
das Magazin zwei Teilmagazine (20,21) umfasst, nämlich
- ein Stechelement-Teilmagazin (20) mit Stechelementkammern (22), die jeweils ein Stechelement (24) enthalten und
- ein Analyseelement-Teilmagazin (21) mit Analyseelementkammern (23), die jeweils ein Analyseelement (25) enthalten.
in der Funktionsposition des Magazins (3) beide Teilmagazine (20,21) in eine Relativposition gebracht werden können, in der jeweils eine Stechelementaustrittsöffnung (33) einer Stechelementkammer (22) und eine Stecheiementeintrittsöffnung (32) einer Analyseelementkammer (23) derartig fluchtend benachbart sind, dass ein Stechelement (24) aus der Stechelemenfikammer (22) durch deren Stechelementaustrittsöffnung (33) und durch die Stechelementeintrittsöffnung (32) der benachbarten Analyseelementkammer (23) in die Analyseelementkammer (23) bewegt werden kann, und
die Stechelementeintrittsöffnung (32) der Analyseelementkammer (23) mittels einer Verschtussfolie (28) verschlossen ist und vor oder während der Stechbewegung des Stechelementes (24) aus der Stechelementkammer (22) in die Analyseelementkammer (23) geöffnet wird,
**dadurch gekennzeichnet, dass**
der Bewegungsweg des Stechelements (24) eine Übergabeposition innerhalb der Kammer des Magazins (3) einschließt, in der die Probenübergabezone (42) des Stechelements (24) der Probenkontaktzone (48) des Analyseelements (25) benachbart ist, um eine Fluidverbindung zur Übertragung einer Körperflüssigkeüitsprobe von dem Stechelement (24) an das Analyseelement (25) herzustellen.

2. Magazin für ein Analysegerät, vorzugsweise als Bestandteil eines Analysesystems nach Anspruch 1, mit Kammern enthaltend
- Analyseelemente (25) mit einer Probenkontaktzone (48) und einem mindestens ein Reagenz enthaltenden Reagenzsystem, dessen Reaktion mit der Körperflüssigkeit zu einer messbaren Änderung einer Messgröße führt,
- Stechelemente (24) mit einer Spitze (37) zum Einstechen in die Haut
mit einem Kapillarkanal (41), der eine Fluidverbindung zwischen der Spitze (37) und einer Probenübergabezone (42) des Stechelementes (24) bildet,
wobei
das Magazin (3) dazu ausgebildet ist, in einer Halterung (8) eines Analysegeräts (2) derartig aufgenommen zu werden, dass jeweils ein Stechelement (24) in einer der Kammern des Magazins (3) von einem Stechantrieb (5) des Analysegerätes (2) auf einem Bewegungsweg, der eine Vortriebsphase in Einstichrichtung umfasst, bewegt werden kann, um eine Stechbewegung auszuführen,
das Magazin (3) zwei Teilmagazine (20,21) umfasst, nämlich
- ein Stechelement-Teilmagazin (20) mit Stechelementkammern (22), die jeweils ein Stechelement (24) enthalten und
- ein Analyseelement-Teilmagazin (21) mit Analyseelementkammern (23), die jeweils ein Analyseelement (25) enthalten,
beide Teilmagazine (20,21) relativ zueinander so angeordnet sind oder angeordnet werden können, dass jeweils eine Stechelementaustrittsöffnung (33) einer Stechelementkammer (22) und eine Stechelementeintrittsöffnung (32) einer Analyseelementkammer (23) derartig fluchtend benachbart sind, dass ein Stechelement (24) aus der Stechelementkammer (22) durch deren Stechelementaustrittsöffnung (33) und durch die Stechelementeintrittsöffnung (32) der benachbarten Analyseelementkammer (23) in die Analyseelementkammer (23) bewegt werden kann, und
die Stechelementeintrittsöffnung (32) der Analyseelementkammer (23) mittels einer Verschlussfolie (28) verschlossen ist, so angeordnet und ausgebildet ist, dass sie die vor oder während der Stechbewegung des Stechelementes (24) aus der Stechelementkammer (22) in die Analyseelementkammer (23) geöffnet werden kann, und der Bewegungsweg des Stechelements (24) eine Übergabeposition innerhalb der Kammer des Magazins (3) einschließt, in der die Probenübergabezone (42) des Stechelements (24) der Probenkontaktzone (48) des Analyseelements (25) benachbart ist, um eine Fluidverbindung zur Übertragung einer Körperflüssigkeitsprobe von dem Stechelement (24) an das Analyseelement (25) herzustellen.

3. Analysesystem nach Anspruch 1 oder Magazin nach Anspruch 2, **dadurch gekennzeichnet, dass** die Stechelementaustrittsöffnung (33) der Stechelementkammer (28) mittels einer Verschlussfolie (28a) verschlossen ist.

4. Analysesystem oder Magazin nach einem der vorhergehenden Ansprüche, **dadurch gekennzeichnet, dass** das Analyseelement (25) in der Analyseelementkammer (23) in einer in Einstichrichtung festen Fluidtransferposition positioniert ist.

5. Analysesystem oder Magazin nach einem der vorhergehenden Ansprüche , **dadurch gekennzeichnet, dass** der Fluidkontakt durch eine quer zu der Einstichrichtung orientierten Relativbewegung zwischen dem Stechelement (24) und dem Analyseelement (25) hergestellt wird.

6. Analysesystem oder Magazin nach Anspruch 5, **dadurch gekennzeichnet, dass** die Probenkontaktzone (48) des Analyseelementes (25) eine in Einstichrichtung orientierte Fläche (49) einschließt und der Fluidkontakt durch Kontaktierung der Fläche (49) der Probenkontaktzone (48) des Analyseelementes (25) mit der Probenübergabezone (42) des Stechelementes (24) hergestellt wird.

7. Analysesystem oder Magazin nach Anspruch 5 oder 6, **dadurch gekennzeichnet, dass** die Relativbewegung zwischen dem Stechelement (24) und dem Analyseelement (25) dadurch bewirkt wird, dass ein Andruckelement (52), welches quer zu der Einstichrichtung beweglich ist, gegen das in der Übergabeposition befindliche Stechelement (24) gedrückt wird.

8. Analysesystem oder Magazin nach Anspruch 7, **dadurch gekennzeichnet, dass** das Andruckelement (52) zwischen dem Stechelement-Teilmagazin (20) und dem Analyseelement-Teilmagazin (21) positioniert ist.

9. Analysesystem oder Magazin nach einem der vorhergehenden Ansprüche, **dadurch gekennzeichnet, dass** das Magazin (3) ein Trommelmagazin (13) ist.

10. Magazin nach einem der Ansprüche 5 bis 10, **dadurch gekennzeichnet, dass** sich der Kapillarkanal (41) des Stechelements (24) über dessen Probenübergabezone (42) hinaus in eine Probenüberschusszone (43) erstreckt.

11. Magazin nach Anspruch 2, **dadurch gekennzeichnet, dass**
das Analyseelement-Teilmagazin (21) einen transparenten Bereich (73) aufweist,
das Analyseelement (25) in dem Analyseelement-Teilmagazin (21) derart relativ zu dem transparenten Bereich (73) angeordnet ist, dass mittels einer photoelektrischen Messeinheit (7), die außerhalb des Magazins (3) positioniert ist, eine Änderung der photometrisch messbaren Messgröße in dem Analyseelement (25) gemessen werden kann.

12. Magazin nach Anspruch 11, **dadurch gekennzeichnet, dass** das Magazin dazu ausgebildet ist, in eine Funktionsposition bewegt zu werden, in der ein Stechelement (24) in einer der Kammern von einem Stechantrieb (5) eines Analysegerätes (2) bewegt werden kann, um eine Stechbewegung auszuführen, und
der transparente Bereich (73) des Analyseelement-Teilmagazins (21) derart angeordnet ist, dass er in der Funktionsposition vor einer Optik der photoelektrischen Messeinheit (7) positioniert ist.

13. Analysesystem oder Magazin nach Anspruch 12, **dadurch gekennzeichnet, dass** an dem Analyseelement-Teilmagazin (21) ein Positionierelement (74) angeordnet ist, und
die photoelektrische Messeinheit (7) eine Positionierkontur (75) aufweist, die mit dem Positionierelement (74) korrespondiert und derart zusammenwirkt, dass eine exakte Positionierung des Analyseelement-Teilmagazins (21), insbesondere des transparenten Bereichs (73), relativ zu der Messeinheit (7) ermöglicht wird.

14. Verfahren zur Herstellung eines zweiteiligen Magazins nach Anspruch 2 mit Kammern,
wobei das Magazin (3) zwei Teilmagazine (20,21) umfasst, nämlich ein StechelementTeilmagazin (20) mit Stechelementkammem (22), die jeweils ein Stechelement (24) enthalten und
ein Analyseelement-Teilmagazin (21) mit Analyseelementkammem (23), die jeweils ein Analyseelement (25) enthalten,
wobei zu Beginn des Herstellungsprozesses die beiden Teilmagazine 20,21 getrennt voneinander sind,
**gekennzeichnet durch** die folgenden Schritte:
a) Bestücken des Stechelement-Teilmagazins (20) mit Stechelementen (24),
b) Verschließen einer Stechalementaustrittsöffnung (33) jeder Stechelementkammer (22) mit einer Verschlussfolie (28,28a),
c) Verschließen einer Verbindungselementeintrittsöffnung (34) jeder Stechelementkammer (22) mit einer Verschlussfolie (36),
d) Sterilisieren der Stechelemente (24) in dem Stechelement-Teilmagazin (20),
e) Verbinden der beiden Teilmagazine (20,21),
f) Verschließen einer Stechelementeintrittsöffnung (32) jeder Analyseelementkammer (23) des Analyseelement-Teilmagazins mit einer Verschlussfolie (28,28a),
g) Bestücken des Analyseelement-Teilmagazins (21) mit Analyseelement (25),
h) Verschließen einer Magazinauslassöffnung (31) jeder Analyseelementkammer (23) des Analyseelement-Teilmagazins (21) mit einer Folie (39).

## Claims

1. Analysis system for determining an analyte in a body fluid sample acquired through a puncture in the skin, comprising
a magazine (3) having chambers, containing
- analysis elements (25) having a sample contact zone (48) and a reagent system containing at least one reagent, whose reaction with the body fluid results in a measurable change of a measuring variable,
- puncturing elements (24) with a tip (37) for piercing into the skin having a capillary channel (41), which forms a fluid connection between the tip (37) and a sample transfer zone (42) of the puncturing element (24),
a reusable analysis instrument (2) having
- a puncturing drive (5) for driving a puncture movement of a puncturing element (24) on a movement path, which comprises a propulsion phase in the puncturing direction and, after reaching a reversal point of the puncture movement, a retraction phase opposite to the puncturing direction,
- a mounting (8) for receiving the magazine (3) in such a manner that one chamber of the magazine (3) at a time is located in a functional position, in which a puncturing element (24) in the chamber can be moved by the puncturing drive (5), and
- a measuring and evaluation apparatus (6) for measuring the measurable change of a measuring variable and for determining a desired analysis result,
wherein
the magazine comprises two partial magazines (20, 21), namely
- a puncturing element partial magazine (20) having puncturing element chambers (22), which each contain one puncturing element (24), and
- an analysis element partial magazine (21) having analysis element chambers (23), which each contain one analysis element (25),
in the functional position of the magazine (3), both partial magazines (20, 21) can be brought into a relative position, in which a puncturing element exit opening (33) of a puncturing element chamber (22) and a puncturing element entry opening (32) of an analysis chamber (23) are adjacent to one another and aligned in such a manner that a puncturing element (24) can be moved from the puncturing element chamber (22) through its puncturing element exit opening (33) and through the puncturing element entry opening (32) of the adjacent analysis element chamber (23) into the analysis element chamber (23), and
the puncturing element entry opening (32) of the analysis element chamber (23) is sealed by means of a sealing film (28) and will be opened before or during the puncture movement of the puncturing element (24) from the puncturing element chamber (22) into the analysis element chamber (23),
**characterized in that**
the movement path of the puncturing element (24) includes a transfer position inside the chamber of the magazine (3), in which the sample transfer zone (42) of the puncturing element (24) is adjacent to the sample contact zone (48) of the analysis element (25), for generating a fluid connection for the transfer of a body fluid sample from the puncturing element (24) to the analysis element (25).

2. Magazine for an analysis instrument, preferably as a component of an analysis system according to claim 1, having chambers containing
- analysis elements (25) having a sample contact zone (48) and a re-agent system containing at least one reagent, whose reaction with the body fluid results in a measurable change of a measuring variable,
- puncturing elements (24) with a tip (37) for piercing into the skin having a capillary channel (41), which forms a fluid connection between the tip (37) and a sample transfer zone (42) of the puncturing element (24),
wherein
the magazine (3) is implemented for being received in a mounting (8) of an analysis instrument (2) in such a manner that one puncturing element (24) at a time in one of the chambers of the magazine (3) can be moved by a puncturing drive (5) of the analysis instrument (2) on a movement path, which comprises a propulsion phase in the puncturing direction, in order to execute a puncture movement,
the magazine (3) comprises two partial magazines (20, 21), namely
- a puncturing element partial magazine (20) having puncturing element chambers (22), which each contain one puncturing element (24), and
- an analysis element partial magazine (21) having analysis element chambers (23), which each contain one analysis element (25),
both partial magazines (20, 21) are located or are able to be located relative to one another so that one puncturing element exit opening (33) of one puncturing element chamber (22) and one puncturing element entry opening (32) of one analysis element chamber (23) are adjacent to one another and aligned in such a manner that one puncturing element (24) can be moved from the puncturing element chamber (22) through its puncturing element exit opening (33) and through the puncturing element entry opening (32) of the adjacent analysis element chamber (23) into the analysis element chamber (23), and
the puncturing element entry opening (32) of the analysis element chamber (23) is sealed by means of a sealing film (28), which is positioned and implemented so that it can be opened before or during the puncture movement of the puncturing element (24) from the puncturing element chamber (22) into the analysis element chamber (23), and
the movement path of the puncturing element (24) includes a transfer position inside the chamber of the magazine (3), in which the sample transfer zone (42) of the puncturing element (24) is adjacent to the sample contact zone (48) of the analysis element (25), for generating a fluid connection for the transfer of a body fluid sample from the puncturing element (24) to the analysis element (25).

3. Analysis system according to claim 1 or the magazine according to claim 2, **characterized in that** the puncturing element exit opening (33) of the puncturing element chamber (28) is sealed by means of a sealing film (28a).

4. Analysis system or magazine according to any one of the preceding claims, **characterized in that** the analysis element (25) is positioned in the analysis element chamber (23) in a fluid transfer position which is fixed in the puncturing direction.

5. Analysis system or magazine according to any one of the preceding claims, **characterized in that** the fluid contact is produced by a relative movement, which is oriented transversely to the puncturing direction, between the puncturing element (24) and the analysis element (25).

6. Analysis system or magazine according to claim 5, **characterized in that** the sample contact zone (48) of the analysis element (25) includes a surface (49) oriented in the puncturing direction, and the fluid contact is produced by contacting the surface (49) of the sample contact zone (48) of the analysis element (25) with the sample transfer zone (42) of the puncturing element (24).

7. Analysis system or magazine according to claim 5 or 6, **characterized in that** the relative movement between the puncturing element (24) and the analysis element (25) is caused **in that** a contact pressure element (52), which is movable transversely to the puncturing direction, is pressed against the puncturing element (24) located in the transfer position.

8. Analysis system or magazine according to claim 7, **characterized in that** the contact pressure element (52) is positioned between the puncturing element partial magazine (20) and the analysis element partial magazine (21).

9. Analysis system or magazine according to any one of the preceding claims, **characterized in that** the magazine (3) is a drum magazine (13).

10. Magazine according to any one of claims 5 to 10, **characterized in that** the capillary channel (41) of the puncturing element (24) extends beyond its sample transfer zone (42) into a sample excess zone (43).

11. Magazine according to claim 2, **characterized in that**
the analysis element partial magazine (21) has a transparent area (73),
the analysis element (25) is positioned in the analysis element partial magazine (21) relative to the transparent area (73) in such a manner that a change of the photometrically measurable measuring variable in the analysis element (25) can be measured by means of a photoelectric measuring unit (7), which is positioned outside the magazine (3).

12. Magazine according to claim 11, **characterized in that** the magazine is adapted for being moved into a functional position, in which a puncturing element (24) in one of the chambers can be moved by a puncturing drive (5) of an analysis instrument (2) in order to execute a puncture movement, and
the transparent area (73) of the analysis element partial magazine (21) is positioned in such a manner that it is positioned in the functional position in front of an optic of the photoelectric measuring unit (7).

13. Analysis system or magazine according to claim 12, **characterized in that** a positioning element (74) is positioned on the analysis element partial magazine (21), and
the photoelectric measuring unit (7) has a positioning contour (75), which corresponds to the positioning element (74) and cooperates therewith in such a manner that exact positioning of the analysis element partial magazine (21), in particular of the transparent area (73), relative to the measuring unit (7) is made possible.

14. Method for producing a two-part magazine according to claim 2, having chambers,
the magazine (3) comprising two partial magazines (20, 21), namely a puncturing element partial magazine (20) having puncturing element chambers (22), which each contain one puncturing element (24), and an analysis element partial magazine (21) having analysis element chambers (23), which each contain one analysis element (25),
wherein the two partial magazines (20, 21) being separate from one another at the beginning of the production process,
**characterized by** the following steps:
a) equipping the puncturing element partial magazine (20) with puncturing elements (24),
b) sealing a puncturing element exit opening (33) of each puncturing element chamber (22) by means of a sealing film (28, 28a),
c) sealing a connection element entry opening (34) of each puncturing element chamber (22) by means of a sealing film (36),
d) sterilizing the puncturing elements (24) in the puncturing element partial magazine (20),
e) connecting the two partial magazines (20,21),
f) sealing a puncturing element entry opening (32) of each analysis element chamber (23) of the analysis element partial magazine by means of a sealing film (28, 28a),
g) equipping the analysis element partial magazine (21) with analysis elements (25),
h) sealing a magazine outlet opening (31) of each analysis element chamber (23) of the analysis element partial magazine (21) by means of a film (39).

## Revendications

1. Système d'analyse destiné à la détermination d'un analyte dans un échantillon de liquide corporel obtenu en piquant la peau, comprenant :
une cartouche (3) dotée de chambres, contenant
- des éléments d'analyse (25) avec une zone de contact d'échantillon (48) et un système de réactif contenant au moins un réactif, dont la réaction avec le liquide corporel conduit à une modification mesurable d'une grandeur de mesure,
- des éléments de piqûre (24) munis d'une pointe (37) pour piquer la peau,
avec un canal capillaire (41) qui établit une communication de fluide entre la pointe (37) et une zone de transfert d'échantillon (42) de l'élément de piqûre (24),
un appareil d'analyse réutilisable (2) comprenant :
- une commande de piqûre (5) pour commander un mouvement de piqûre d'un élément de piqûre (24) sur un trajet de mouvement qui comporte une phase d'avancement dans le sens de la piqûre et, après avoir atteint un point d'inversion du mouvement de piqûre, une phase de recul dans le sens inverse de la piqûre,
- un support (8) servant à recevoir la cartouche (3) de façon que respectivement une chambre de la cartouche (3) se trouve dans une position fonctionnelle dans laquelle un élément de piqûre (24) peut être déplacé dans la chambre par la commande de piqûre (5), et
- un dispositif de mesure et d'évaluation (6) pour mesurer la modification mesurable d'une grandeur de mesure et pour obtenir un résultat d'analyse souhaité,
la cartouche comprenant deux cartouches partielles (20, 21), à savoir
- une cartouche partielle d'éléments de piqûre (20), dotée de chambres d'élément de piqûre (22) contenant chacune un élément de piqûre (24), et
- une cartouche partielle d'éléments d'analyse (21), dotée de chambres d'élément d'analyse (23) contenant chacune un élément d'analyse (25),
les deux cartouches partielles (20, 21), lorsque la cartouche (3) est en position fonctionnelle, pouvant être amenées dans une position relative dans laquelle respectivement un orifice de sortie d'élément de piqûre (33) d'une chambre d'élément de piqûre (22) et un orifice d'entrée d'élément de piqûre (32) d'une chambre d'élément d'analyse (23) sont adjacents et alignés de telle sorte qu'un élément de piqûre (24) peut être déplacé hors de la chambre d'élément de piqûre (22), à travers l'orifice de sortie d'élément de piqûre (33) de celle-ci et à travers l'orifice d'entrée d'élément de piqûre (32) de la chambre d'élément d'analyse (23) adjacente, dans ladite chambre d'élément d'analyse (23), et
l'orifice d'entrée d'élément de piqûre (32) de la chambre d'élément d'analyse (23) étant obturé par un opercule (28) et s'ouvrant avant ou pendant le mouvement de piqûre de l'élément de piqûre (24) hors de la chambre d'élément de piqûre (22) dans la chambre d'élément d'analyse (23),
**caractérisé en ce que**
le trajet de mouvement de l'élément de piqûre (24) englobe une position de transfert à l'intérieur de la chambre de la cartouche (3), dans laquelle la zone de transfert d'échantillon (42) de l'élément de piqûre (24) est adjacente à la zone de contact d'échantillon (48) de l'élément d'analyse (25) afin d'établir une communication de fluide permettant de transférer un échantillon de liquide corporel de l'élément de piqûre (24) sur l'élément d'analyse (25).

2. Cartouche pour un appareil d'analyse, de préférence comme élément d'un système d'analyse selon la revendication 1, dotée de chambres contenant
- des éléments d'analyse (25) avec une zone de contact d'échantillon (48) et un système de réactif contenant au moins un réactif, dont la réaction avec le liquide corporel conduit à une modification mesurable d'une grandeur de mesure,
- des éléments de piqûre (24) munis d'une pointe (37) pour piquer la peau, avec un canal capillaire (41) qui établit une communication de fluide entre la pointe (37) et une zone de transfert d'échantillon (42) de l'élément de piqûre (24),
la cartouche (3) étant conçue pour être reçue dans un support (8) d'un appareil d'analyse (2) de telle sorte que respectivement un élément de piqûre (24) peut être déplacé dans l'une des chambres de la cartouche (3) par une commande de piqûre (5) de l'appareil d'analyse (2) sur un trajet de mouvement comportant une phase d'avancement dans le sens de la piqûre, afin d'effectuer un mouvement de piqûre,
la cartouche (3) comprenant deux cartouches partielles (20, 21), à savoir
- une cartouche partielle d'éléments de piqûre (20), dotée de chambres d'élément de piqûre (22) contenant chacune un élément de piqûre (24), et
- une cartouche partielle d'éléments d'analyse (21), dotée de chambres d'élément d'analyse (23) contenant chacune un élément d'analyse (25),
les deux cartouches partielles (20, 21) étant ou pouvant être disposés l'une par rapport à l'autre de façon que respectivement un orifice de sortie d'élément de piqûre (33) d'une chambre d'élément de piqûre (22) et un orifice d'entrée d'élément de piqûre (32) d'une chambre d'élément d'analyse (23) soient adjacents et alignés de telle sorte qu'un élément de piqûre (24) peut être déplacé hors de la chambre d'élément de piqûre (22), à travers l'orifice de sortie d'élément de piqûre (33) de celle-ci et à travers l'orifice d'entrée d'élément de piqûre (32) de la chambre d'élément d'analyse (23) adjacente, dans ladite chambre d'élément d'analyse (23), et
l'orifice d'entrée d'élément de piqûre (32) de la chambre d'élément d'analyse (23) étant obturé par un opercule (28) et étant disposé et conçu de manière qu'il puisse s'ouvrir avant ou pendant le mouvement de piqûre de l'élément de piqûre (24) hors de la chambre d'élément de piqûre (22) dans la chambre d'élément d'analyse (23), et
le trajet de mouvement de l'élément de piqûre (24) englobant une position de transfert à l'intérieur de la chambre de la cartouche (3), dans laquelle la zone de transfert d'échantillon (42) de l'élément de piqûre (24) est adjacente à la zone de contact d'échantillon (48) de l'élément d'analyse (25) afin d'établir une communication de fluide permettant de transférer un échantillon de liquide corporel de l'élément de piqûre (24) sur l'élément d'analyse (25).

3. Système d'analyse selon la revendication 1 ou cartouche selon la revendication 2, caractérisé(e) en ce que l'orifice de sortie d'élément de piqûre (33) de la chambre d'élément de piqûre (28) est obturé par un opercule (28a).

4. Système d'analyse ou cartouche selon l'une des revendications précédentes, caractérisé(e) en ce que l'élément d'analyse (25) est positionné dans la chambre d'élément d'analyse (23) dans une position de transfert de fluide fixe dans le sens de la piqûre.

5. Système d'analyse ou cartouche selon l'une des revendications précédentes, caractérisé(e) en ce que le contact de fluide est établi par un mouvement relatif, orienté transversalement au sens de la piqûre, entre l'élément de piqûre (24) et l'élément d'analyse (25).

6. Système d'analyse ou cartouche selon la revendication 5, caractérisé(e) en ce que la zone de contact d'échantillon (48) de l'élément d'analyse (25) englobe une surface (49) orientée dans le sens de la piqûre, et le contact de fluide est établi par une mise en contact de ladite surface (49) de la zone de contact d'échantillon (48) de l'élément d'analyse (25) avec la zone de transfert d'échantillon (42) de l'élément de piqûre (28).

7. Système d'analyse ou cartouche selon la revendication 5 ou 6, caractérisé(e) en ce que le mouvement relatif entre l'élément de piqûre (24) et l'élément d'analyse (25) est provoqué par le fait qu'un élément de pression (52), mobile transversalement par rapport au sens de la piqûre, est pressé contre l'élément de piqûre (24) se trouvant en position de transfert.

8. Système d'analyse ou cartouche selon la revendication 7, caractérisé(e) en ce que l'élément de pression (52) est positionné entre la cartouche partielle d'éléments de piqûre (20) et la cartouche partielle d'éléments d'analyse (21).

9. Système d'analyse ou cartouche selon l'une des revendications précédentes, caractérisé(e) en ce que la cartouche (3) est une cartouche à tambour (13).

10. Cartouche selon l'une des revendications 5 à 10, **caractérisée en ce que** le canal capillaire (41) de l'élément de piqûre (24) s'étend au-delà de la zone de transfert d'échantillon (42) de celui-ci jusque dans une zone d'excédent d'échantillon (43).

11. Cartouche selon la revendication 2, **caractérisée en ce que**
la cartouche d'éléments d'analyse (21) présente une zone transparente (73),
l'élément d'analyse (25) est disposé dans la cartouche d'éléments d'analyse (21) par rapport à ladite zone transparente (73) de façon à pouvoir mesurer dans l'élément d'analyse (25) une modification de la grandeur de mesure, mesurable par photométrie, au moyen d'une unité de mesure photoélectrique (7) positionnée à l'extérieur de la cartouche (3).

12. Cartouche selon la revendication 11, **caractérisée en ce que** la cartouche est conçue pour être amenée dans une position fonctionnelle dans laquelle un élément de piqûre (24) peut être déplacé dans l'une des chambres par une commande de piqûre (5) de l'appareil d'analyse (2) afin d'effectuer un mouvement de piqûre, et
la zone transparente (73) de la cartouche partielle d'éléments d'analyse (25) est disposée de façon à être positionnée en position fonctionnelle devant une optique de l'unité de mesure photoélectrique (7).

13. Système d'analyse ou cartouche selon la revendication 12, caractérisé(e) en ce qu'un élément de positionnement (74) est disposé sur la cartouche partielle d'éléments d'analyse (21), et
l'unité de mesure photoélectrique (7) présente un contour de positionnement (75) correspondant et coopérant avec ledit élément de positionnement (74), permettant ainsi un positionnement précis de la cartouche partielle d'éléments d'analyse (21), notamment de la zone transparente (73), par rapport à ladite unité de mesure (7).

14. Procédé de fabrication d'une cartouche en deux parties selon la revendication 2 dotée de chambres,
la cartouche (3) comprenant deux cartouches partielles (20, 21), à savoir une cartouche partielle d'éléments de piqûre (20) avec des chambres d'élément de piqûre (22) contenant chacune un élément de piqûre (24), et une cartouche partielle d'éléments d'analyse (21) avec des chambres d'élément d'analyse (23) contenant chacune un élément d'analyse (25),
les deux cartouches partielles (20, 21) étant séparées au début du processus de fabrication,
**caractérisé par** les étapes suivantes :
a) équipement de la cartouche partielle par des éléments de piqûre (24)
b) obturation d'un orifice de sortie d'élément de piqûre (33) de chaque chambre d'élément de piqûre (22) avec un opercule (28, 28a),
c) obturation d'un orifice d'entrée d'élément de liaison (34) de chaque chambre d'élément de piqûre (22) avec un opercule (36),
d) stérilisation des éléments de piqûre (24) dans la cartouche partielle d'éléments de piqûre (20),
e) assemblage des deux cartouches partielles (20, 21),
f) obturation d'un orifice d'entrée d'élément de piqûre (32) de chaque chambre d'élément d'analyse (23) de la cartouche partielle d'éléments d'analyse avec un opercule (28, 28a),
g) équipage de la cartouche partielle d'éléments d'analyse (21) avec éléments d'analyse (25),
h) obturation d'un orifice de sortie de cartouche (31) de chaque chambre d'élément d'analyse (23) de la cartouche partielle d'éléments d'analyse (21) avec un opercule (39).
